(19) Europäisches Patentamt
European Patent Office
Office européen des brevets

(11) EP 3 628 666 A1

(12) **EUROPEAN PATENT APPLICATION**

(43) Date of publication:
**01.04.2020 Bulletin 2020/14**

(51) Int Cl.:
***C07D 277/64*** *(2006.01)* ***A61P 25/00*** *(2006.01)*
***A61P 31/04*** *(2006.01)* ***A61K 31/428*** *(2006.01)*

(21) Application number: **18290106.6**

(22) Date of filing: **25.09.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Antabio SAS**
**31670 Labège (FR)**

(72) Inventor: **The designation of the inventor has not yet been filed**

(74) Representative: **J A Kemp LLP**
**14 South Square**
**Gray's Inn**
**London WC1R 5JJ (GB)**

(54) **INDANE DERIVATIVES FOR USE IN THE TREATMENT OF BACTERIAL INFECTION**

(57) The invention relates to a compound which is an indane according to Formula (I), or a pharmaceutically acceptable salt thereof,

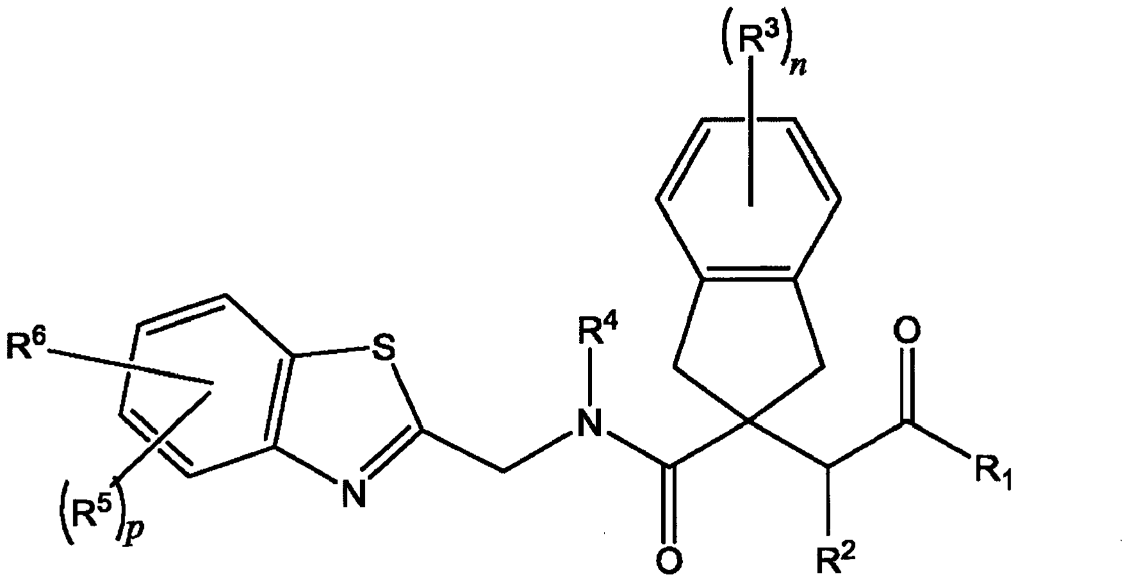

[FORMULA (I)]

wherein $R^1$, $R^2$, $R^3$, $R^4$, $R^5$, $R^6$, *n* and p are as defined herein. The compounds are useful in the treatment of antibacterial infection either as stand alone antibiotics, or in combination with further antibiotics.

EP 3 628 666 A1

## Description

### Field of the Invention

The present invention relates to compounds which find use in the prevention or treatment of bacterial infection. The invention also provides such compounds *per se* and pharmaceutical compositions comprising such compounds.

### Background

Cystic fibrosis (CF) is a life-threatening disease affecting approximately 70,000 sufferers worldwide. CF is the most common lethal, hereditary disease in Caucasian populations, resulting from mutations in the cystic fibrosis transmembrane conductance regulator (CFTR) gene. The prevalence of CF in Europe is 1 in every 2,000-3,000 live births, and in North America is about 1 in every 3,500 births. In the UK there are approximately 9,800 people with CF.

The organs of individuals with CF typically have significantly thickened secretions. This in turn can lead to a range of pathological problems. For instance, individuals with CF typically have impaired ciliary clearance, and the lungs of such individuals are typically colonized and infected by bacteria from an early age. Such bacteria include *Staphylococcus aureus, Haemophilus influenza, Pseudomonas aeruginosa* and *Burkholderia cepacia. Pseudomonas aeruginosa* (PA) is the most common cause of chronic lung infection in individuals with CF, and chronic infection with PA is found in 9% of pre-school children, 32% of 10-15 year olds and the majority (between 59% and 80%) of adults with CF, leading to progressive lung damage and early death.

As the lung of the individual with CF is colonised by PA, the growth pattern of the bacteria changes and its capacity for survival improves. In chronic infection, PA bacteria on mucosal and epithelial surfaces, or in sputum, form biofilms as well as producing large quantities of alginate (the so-called mucoid phenotype) which reduce the effectiveness of phagocytosis and antibiotic therapy. This leads to chronic colonisation of the lung by PA that is not cleared by conventional antibiotic therapy.

Antibiotics are a broad range of substances exhibiting anti-bacterial activity. A large number of antibiotic compounds are known and have been shown to exhibit antibacterial activity against a wide range of bacteria. However, currently available antibiotics are incapable of controlling some bacterial infections. This is because the target bacteria have acquired antibiotic resistance, for example via horizontal gene transfer or because the target bacteria are found in a state in which the efficacy of antibiotics which would otherwise be highly active is reduced. One such state is a bacterial biofilm.

Bacteria in biofilms are enclosed in a self-produced extracellular biopolymer matrix, which may include polysaccharides, proteins and DNA. Bacteria in biofilms typically exhibit different properties from free-living bacteria of the same species. Such properties typically include increased resistance to antibiotics and detergents and increased lateral gene transfer. For example, bacteria in biofilms typically display up to 1,000-fold higher tolerance to antibiotic challenge than their single cell, planktonic (free-living) counterparts.

This limitation in the efficacy of antibacterial compounds is especially important for individuals who through immunodeficiency or other diseases or conditions cannot adequately combat bacterial infection. Such individuals include those suffering from cystic fibrosis.

CF patients who are colonised with PA show also a more rapid decline in lung function, faster decline in chest radiograph score, poor weight gain, increased hospitalisation rates and an increased need for antibiotic therapy. Median survival is reduced and mortality increased (2.6x risk of death). Most disease-related morbidity and mortality in CF is caused by progressive lung disease as a result of bacterial infection and airway inflammation, primarily associated with the effects of chronic PA lung infection and the persistence of PA biofilms. Despite intensive antibiotic treatment, adaptive mechanisms such as biofilm formation allow PA to resist both immune and antibiotic pressures, leading to recurrent exacerbations and respiratory failure.

Pathogenic bacteria such as PA are not only of importance in the context of CF. For example, the opportunistic pathogen PA can also cause septic shock, particularly in neutropenic patients, and can be responsible for infections of the urinary tract, the gastrointestinal network and skin and soft tissues. PA is also a frequent coloniser of medical devices such as catheters, nebulizers, and the like.

Accordingly, there is a clear need for new antibiotic compounds and compositions and adjuvant therapies for treating bacterial infection.

### Summary of the Invention

The inventors have surprisingly found that compounds of Formula (I) are potent inhibitors of the *Pseudomonas aeruginosa*-derived elastase enzyme LasB, which is important in *Pseudomonas aeruginosa* pathogenesis and persistence through biofilm formation.

LasB is implicated in bacterial disease pathology, since secreted LasB degrades many host immune proteins and causes tissue damage. LasB, also known as pseudolysin, is massively secreted into the environment of the producer organism where it is able to proteolytically attack numerous host immune proteins (e.g. immunoglobulins, cytokines, SP-A, antimicrobial peptides (e.g. Trappin 2)) and tissue proteins (e.g. elastin). There are no mammalian homologues of LasB. The ability of LasB to attack host proteins contributes to immune evasion (e.g. avoidance of SP-A mediated phagocytosis, and degradation of immunoglobulin, degradation of antimicrobial peptides (e.g. Trappin 2)) whilst promoting tissue invasion and long term colonization. Inhibition of LasB therefore better equips the host to deal with immune attack.

LasB also has an important internal role within the bacterial cell cleaving nucleoside diphosphate kinase (NDK) to a smaller active form. Active form of NDK leads to increased GTP levels within the cell, increasing production of alginate. Alginate is a polysaccharide which is a major component of the extracellular biofilm matrix and which is required for swarming motility. Those two virulence phenotypes are associated with bacterial persistence in response to immune and antibiotic pressures. LasB activity has also been shown to upregulate rhamnolipid production, which is necessary for biofilm formation/ maturation. Accordingly, inhibition of LasB assists impairment of biofilm formation and disruption of the established biofilm. This in turn is believed to better enable antibiotics currently in use to deal effectively with infection.

Accordingly, the present invention provides the following aspects:

1. A compound which is an indane according to Formula (I), or a pharmaceutically acceptable salt thereof,

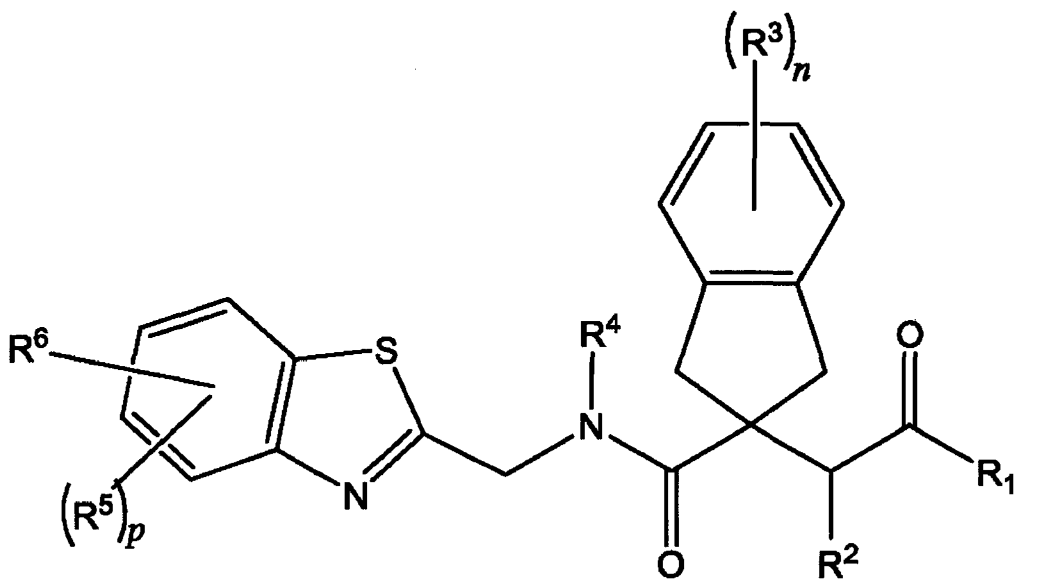

[FORMULA (I)]

wherein

- $R^1$ is selected from:
  - NHOH, -OH, $-OR^{1a}$ and $-OCH_2OC(O)R^{1a}$, wherein $R^{1a}$ is selected from an unsubstituted $C_1$ to $C_4$ alkyl group and phenyl; and
  - where the compound of Formula (I) contains a positively charged nitrogen atom, $R^1$ may be O , such that the compound forms a zwitterion;
- $R^2$ is selected from H and unsubstituted $C_1$ to $C_2$ alkyl;
- each $R^3$ group is independently selected from halogen, -OH, $-NH_2$, methyl and $-CF_3$;
- *n* is an integer from 0 to 4;
- $R^4$ is selected from H and unsubstituted $C_1$ to $C_2$ alkyl;
- $R^6$ is $C_2$ to $C_4$ alkoxy which is unsubstituted or is substituted with a group selected from -OH; $-NR^{10}R^{11}$; $-N^+R^{10}R^{11}R^{12}$; $-OR^{6a}$ and $-NR^{10}R^{6a}$, wherein $R^{6a}$ is a $C_1$ to $C_3$ alkyl group which is unsubstituted or substituted with a group selected from OH; $-NR^{10}R^{11}$; $-N^+R^{10}R^{11}R^{12}$; $-NR^{10}NR^{11}R^{12}$; $-NR^{10}N^+R^{11}R^{12}R^{13}$;

$-N^+R^{10}R^{11}NR^{12}R^{13}$;
$-NR^{10}C(NR^{11})NR^{12}R^{13}$; $-NR^{10}C(N^+R^{11}R^{12})NR^{13}R^{14}$; $-C(NR^{10})NR^{11}R^{12}$; and
$-C(N^+R^{10}R^{11})NR^{12}R^{13}$;

- *p* is 0 or 1;
- $R^5$ is selected from -OMe, -OH, halogen, $-NR^{10}R^{11}$; $-N^+-R^{10}R^{11}R^{12}$, $-CF_3$; and
- $R^{10}$, $R^{11}$, $R^{12}$ $R^{13}$ and $R^{14}$ are independently H or methyl;

with the proviso that the indane of Formula (I) is other than:

2-(2-(((4-ethoxybenzo[d]thiazol-2-yl)methyl)carbamoyl)-2,3-dihydro-1H-inden-2-yl)acetic acid;
2-[2-[(6-ethoxy-1,3-benzothiazol-2-yl)methylcarbamoyl] indan-2-yl]acetic acid;
2-[2-[[6-(2-hydroxyethoxy)-1,3-benzothiazol-2-yl]methylcarbamoyl]indan-2-yl]acetic acid;
2-[2-[[6-[2-(dimethylamino)ethoxy]-1,3-benzothiazol-2-yl]methylcarbamoyl]indan-2-yl]acetic acid;
2-[2-[[6-[2-(trimethylammonio)ethoxy]-1,3-benzothiazol-2-yl]methylcarbamoyl]indan-2-yl]acetate;
2-[2-[[5-[2-(dimethylamino)ethoxy]-1,3-benzothiazol-2-yl]methylcarbamoyl]indan-2-yl]acetic acid;
2-[2-[[5-[2-(trimemylammonio)ethoxy]-1,3-benzothiazol-2-yl]methylcarbamoyl]indan-2-yl]acetate;
2-(2-(((5-(3-(dimethylamino)propoxy)-6-methoxybenzo[d]thiazol-2-yl)methyl)carbamoyl)-5,6-difluoro-2,3-dihydro-1H-inden-2-yl)acetic acid;
2-(5,6-difluoro-2-(((6-methoxy-5-(3-(trimethylammonio)propoxy)benzo[d]thiazol-2-yl)methyl)carbamoyl)-2,3-dihydro-1H-inden-2-yl)acetate; and
2-(2-(((5-(2-(dimethylamino)ethoxy)-6-methoxybenzo[d]thiazol-2-yl)methyl)carbamoyl)-2,3-dihydro-1H-inden-2-yl)acetic acid.

2. A compound according to aspect 1, wherein $R^1$ is selected from -OH and -NHOH, or where the compound of Formula (I) contains a positively charged nitrogen atom, $R^1$ may be O , such that the compound forms a zwitterion.

3. A compound according to aspect 1 or aspect 2, wherein $R^2$ is H.

4. A compound according to aspect 1 or aspect 2, wherein $R^4$ is H.

5. A compound according to any one of the preceding aspects, wherein *n* is an integer from 0 to 2 and each $R^3$ group is halogen, preferably fluorine.

6. A compound according to any one of the preceding aspects, wherein $R^5$ is methoxy.

7. A compound according to any one of the preceding aspects, wherein $R^6$ is $C_2$ to $C_4$ alkoxy which is unsubstituted or is substituted with a group selected from -OH; $-NR^{10}R^{11}$; - $N^+R^{10}R^{11}R^{12}$; and $-OR^{6a}$, wherein $R^{6a}$ is a $C_1$ to $C_3$ alkyl group which is unsubstituted or substituted with a group selected from OH; $-NR^{10}R^{11}$; and $-N^+R^{10}R^{11}R^{12}$.

8. A compound according to any one of the preceding aspects, wherein *p* is 1; and $R^6$ is $C_2$ to $C_4$ alkoxy which is substituted with a group selected from $-NMe_2$; $-N^+(Me)_3$; and $-OR^{6a}$, wherein $R^{6a}$ is a $C_1$ to $C_3$ alkyl group which is unsubstituted or substituted with a group selected from $-NR^{10}R^{11}$; and $-N^+R^{10}R^{11}R^{12}$.

9. A compound according to any one of aspects 1 to 6, wherein $R^6$ is $C_2$ to $C_4$ alkoxy which is substituted with a group selected from $-OR^{6a}$ and $-NR^{10}R^{6a}$, wherein $R^{6a}$ is a $C_1$ to $C_3$ alkyl group which is unsubstituted or substituted with a group selected from OH; $-NR^{10}R^{11}$; $-N^+R^{10}R^{11}R^{12}$; $-NR^{10}R^{11}R^{12}$; $-NR^{10}N^+R^{11}R^{12}R^{13}$; $-N^+R^{10}R^{11}NR^{12}R^{13}$; $-NR^{10}C(NR^{11})NR^{12}R^{13}$; $-NR^{10}C(N^+R^{11}R^{12})NR^{13}R^{14}$; $-C(NR^{10})NR^{11}R^{12}$; and $-C(N^+R^{10}R^{11})NR^{12}R^{13}$.

10. A compound according to any one of aspects 1 to 6, wherein $R^6$ is $C_2$ to $C_4$ alkoxy which is substituted with a group $-OR^{6a}$, wherein $R^{6a}$ is a $C_1$ to $C_3$ alkyl group which is unsubstituted or substituted with a group selected from OH; $-NR^{10}R^{11}$; and $-N^+R^{10}R^{11}R^{12}$.

11. A compound according to aspect 1 which is 2-[5,6-difluoro-2-[[6-methoxy-5-[2-[2-(trimethylammonio)ethoxy]ethoxy]-1,3-benzothiazol-2-yl]methylcarbamoyl]indan-2-yl]acetate or a pharmaceutically acceptable salt thereof.

12. A compound according to aspect 1 which is 2-[2-[[6-methoxy-5-[3-(trimethylammonio)propoxy]-1,3-benzothiazol-2-yl]methylcarbamoyl]indan-2-yl]acetate or a pharmaceutically acceptable salt thereof.

13. A compound according to aspect 1 which is 2-[2-[[6-methoxy-5-[2-[2-(trimethylammonio)ethoxy]ethoxy]-1,3-benzothiazol-2-yl]methylcarbamoyl]indan-2-yl]acetate or a pharmaceutically acceptable salt thereof.

14. A compound according to aspect 1 which is
2-[2-[[6-(3-hydroxypropoxy)-1,3-benzothiazol-2-yl]methylcarbamoyl] indan-2-yl] acetic acid;
2-[2-[(6-propoxy-1,3-benzothiazol-2-yl)methylcarbamoyl]indan-2-yl]acetic acid;
2-[2-[[5-[3-(dimethylamino)propoxy]-6-methoxy-1,3-benzothiazol-2-yl]methylcarbamoyl]indan-2-yl]acetic acid;
2-[5,6-difluoro-2-[[6-methoxy-5-[2-(trimethylammonio)ethoxy]-1,3-benzothiazol-2-yl]methylcarbamoyl]indan-2-yl]acetate;
2-(2-(((5-(4-(dimethylamino)butoxy)-6-methoxybenzo[d]thiazol-2-yl)methyl)carbamoyl)-2,3-dihydro-1H-inden-2-yl)acetic acid;
2-(2-(((6-methoxy-5-(4-(trimethylammonio)butoxy)benzo[d]thiazol-2-yl)methyl)carbamoyl)-2,3-dihydro-1H-inden-2-yl)acetate;
or a pharmaceutically acceptable salt thereof.

15. A pharmaceutical composition comprising (i) a compound according to any one of the preceding aspects and (ii) at least one pharmaceutically acceptable carrier or diluent, and optionally further comprising (iii) an antibiotic agent, wherein preferably the antibiotic agent is selected from tobramycin, neomycin, streptomycin, gentamycin, ceftazidime, ticarcillin, piperacillin, tazobactam, imipenem, meropenem, rifampicin, ciprofloxacin, amikacin, colistin, aztreonam and levofloxacin.

16. A combination of (i) a compound according to any one of aspects 1 to 14 and (ii) an antibiotic agent; wherein preferably the antibiotic agent is selected from tobramycin, neomycin, streptomycin, gentamycin, ceftazidime, ticarcillin, piperacillin, tazobactam, imipenem, meropenem, rifampicin, ciprofloxacin, amikacin, colistin, aztreonam and levofloxacin.

17. A compound according to any one of aspects 1 to 14; a composition according to aspect 15 or a combination according to aspect 16 for use in medicine.

18. A compound according to any one of aspects 1 to 14; a composition according to aspect 15 or a combination according to aspect 16 for use in treating or preventing bacterial infection in a subject.

19. A compound for use, composition for use or combination for use according to aspect 18 wherein the bacterial infection is caused by *Bacillus, Pseudomonas, Staphylococcus, Streptococcus, Listeria, Burkholderia or Escherichia.*

20. A compound for use, composition for use or combination for use according to aspect 18 or 19 which is for use in the treatment or prevention of pneumonia.

21. A compound for use, composition for use or combination for use according to any one of aspects 18 to 20 wherein the subject suffers from cystic fibrosis.

## DETAILED DESCRIPTION OF THE INVENTION

*Definitions*

As used herein, a $C_1$ to $C_4$ alkyl group is a linear or branched alkyl group containing from 1 to 4 carbon atoms. A $C_1$ to $C_4$ alkyl group is often a $C_1$ to $C_3$ alkyl group. Examples of $C_1$ to $C_4$ alkyl groups include methyl, ethyl, n-propyl, iso-propyl, n-butyl, sec-butyl, and tert-butyl. A $C_1$ to $C_3$ alkyl group is typically a $C_1$ to $C_2$ alkyl group. A $C_1$ to $C_2$ alkyl group is methyl or ethyl, typically methyl. For the avoidance of doubt, where two alkyl groups are present, the alkyl groups may be the same or different.

As used herein, an alkoxy group is typically a said alkyl group attached to an oxygen atom. Thus, a $C_2$ to $C_4$ alkoxy group is a $C_2$ to $C_4$ alkyl group attached to an oxygen atom. A $C_1$ to $C_3$ alkoxy group is a $C_1$ to $C_3$ alkyl group attached to an oxygen atom. Examples of $C_2$ to $C_4$ alkoxy groups include ethoxy, n-propyoxy, iso-propoxy, n-butoxy, sec-butoxy, and tert-butoxy. Examples of $C_1$ to $C_3$ alkoxy groups include methoxy, ethoxy n-propyoxy and iso-propoxy. Typically, a $C_1$ to $C_3$ alkoxy group is a $C_1$ to $C_2$ alkoxy group such as a methoxy or ethoxy group. For the avoidance of doubt, where two alkoxy groups are present, the alkoxy groups may be the same or different.

As used herein, a halogen is typically chlorine, fluorine, bromine or iodine and is preferably chlorine, bromine or fluorine, especially chorine or fluorine.

As used herein, a pharmaceutically acceptable salt is a salt with a pharmaceutically acceptable acid or base. Pharmaceutically acceptable acids include both inorganic acids such as hydrochloric, sulphuric, phosphoric, diphosphoric, hydrobromic or nitric acid and organic acids such as oxalic, citric, fumaric, maleic, malic, ascorbic, succinic, tartaric, palmitic, benzoic, acetic, triphenylacetic, methanesulphonic, ethanesulphonic, 1-hydroxy-2-naphthenoic, isethionic, benzenesulphonic or *p*-toluenesulphonic acid. Pharmaceutically acceptable bases include alkali metal (e.g. sodium or potassium), alkali earth metal (e.g. calcium or magnesium) and zinc bases, for example hydroxides, carbonates, and bicarbonates, and organic bases such as alkyl amines, aralkyl (i.e. aryl-substituted alkyl; e.g. benzyl) amines and heterocyclic amines.

Where the compound of Formula (I) contains a positively charged nitrogen atom, the compound may exist as a zwitterion, where $R^1$ is $O^-$, thus leaving a $COO^-$ group. Such compounds may also be provided in the form of a pharmaceutically acceptable salt. Suitable salts include those formed with pharmaceutically acceptable acids, which provide a proton to the $COO^-$ group, and a counter-ion to balance the positive charge on the quaternary nitrogen atom. Suitable pharmaceutically acceptable acids include hydrochloric acid, sulphonic acids including methanesulphonic acid and toluene sulphonic acid, ascorbic acid and citric acid. Hydrochloric acid and sulphonic acids are preferred, in particular hydrochloric acid. Alternatively, zwitterions can be combined with pharmaceutically acceptable bases as mentioned above, for example, alkali metal (e.g. sodium or potassium) and alkali earth metal (e.g. calcium or magnesium) hydroxides.

In Formula (I), the stereochemistry is not limited. In particular, compounds of Formula (I) containing one or more stereocentre (e.g. one or more chiral centre) may be used in enantiomerically or diastereoisomerically pure form, or in the form of a mixture of isomers. Further, for the avoidance of doubt, the compounds of the invention may be used in any tautomeric form. Typically, the agent or substance described herein contains at least 50%, preferably at least 60, 75%, 90% or 95% of a compound according to Formula (I) which is enantiomerically or diasteriomerically pure. Thus, the compound is preferably substantially optically pure.

For the avoidance of doubt, the terms 'indanyl derivative' and 'indane derivative' may be used interchangeably and unless otherwise indicated refer to compounds of the invention, such as compounds of Formula (I).

*Compounds of the Invention*

Typically, $R^1$ is selected from OH, NHOH and $OR^{1a}$, e.g. from OH and $OR^{1a}$, or where the compound of Formula (I) contains a positively charged nitrogen atom, $R^1$ maybe $O^-$, such that the compound forms a zwitterion. $R^{1a}$ is typically an unsubstituted $C_1$ to $C_4$ alkyl group, such as an unsubstituted $C_1$ to $C_2$ alkyl group. More preferably, $R^{1a}$ is methyl or t-butyl.

More preferably, $R^1$ is OH or NHOH, or where the compound of Formula (I) contains a positively charged nitrogen atom, $R^1$ may be $O^-$, such that the compound forms a zwitterion. Still more preferably, $R^1$ is OH, or where the compound of Formula (I) contains a positively charged nitrogen atom, $R^1$ may be $O^-$, such that the compound forms a zwitterion.

Typically, $R^2$ is selected from H and methyl. Most preferably, $R^2$ is H. $R^4$ is typically H or methyl. Preferably, $R^4$ is H. Most preferably, $R^2$ and $R^4$ are independently H or methyl, most preferably they are both H.

Each $R^3$ group is typically independently selected from halogen; and -OH; and $-NH_2$. More preferably, each $R^3$ group is independently selected from halogen (e.g. fluorine or chlorine) and -OH. Yet more preferably each $R^3$ group is halogen, most preferably fluorine. Typically, *n* is an integer from 0 to 2; more preferably *n* is 0 or 1; most preferably *n* is 0. Preferably, where more than one $R^3$ group is present, each $R^3$ is the same.

Preferably, in Formula (I), $R^1$ is selected from OH and NHOH, or where the compound of Formula (I) contains a positively charged nitrogen atom, $R^1$ may be $O^-$, such that a $COO^-$ group is present and the compound forms a zwitterion; $R^2$ is selected from H and methyl; each $R^3$ group is independently selected from halogen (e.g. fluorine or chlorine); and -OH; *n* is an integer from 0 to 2, and $R^4$ is H.

More preferably, in Formula (I), $R^1$ is OH, or where the compound of Formula (I) contains a positively charged nitrogen atom, $R^1$ may be $O^-$, such that a $COO^-$ group is present and the compound forms a zwitterion; $R^2$ is H; each $R^3$ group is independently selected from halogen, preferably fluorine; *n* is an integer from 0 to 2, and $R^4$ is H.

$R^5$ is preferably methoxy.

*p* is 0 or 1, preferably 1.

$R^6$ is $C_2$ to $C_4$ alkoxy, for example ethoxy, n-propoxy or n-butoxy, preferably ethoxy or n-propoxy, each of which may be unsubstituted or substituted.

Typically, $R^6$ is unsubstituted or is substituted with a group selected from -OH; $-NR^{10}R^{11}$; $-N^+R^{10}R^{11}R^{12}$; and $-OR^{6a}$. In one embodiment, $R^6$ is $C_2$ to $C_4$ alkoxy which is substituted with a group selected from -OH; $-NR^{10}R^{11}$; $-N^+R^{10}R^{11}R^{12}$; $-OR^{6a}$ and $-NR^{10}R^{6a}$. Preferably, $R^6$ is $C_2$ to $C_4$ alkoxy which is substituted with a group selected from -OH; $-NR^{10}R^{11}$; $-N^+R^{10}R^{11}R^{12}$; and $-OR^{6a}$. Most preferably, $R^6$ is $C_2$ to $C_4$ alkoxy which is substituted with a group

selected from $-NR^{10}R^{11}$; $-N^+R^{10}R^{11}R^{12}$; and $-OR^{6a}$. Most preferably, $R^6$ is $C_2$ to $C_4$ alkoxy which is substituted with a group $-OR^{6a}$.

$R^{6a}$ is typically a $C_1$ to $C_3$ alkyl group which is unsubstituted or substituted with a group selected from OH; $-NR^{10}R^{11}$; and $-N^+R^{10}R^{11}R^{12}$. Preferably, $R^{6a}$ is a $C_1$ to $C_3$ alkyl group which is unsubstituted or substituted with a group selected from OH; $-NMe_2$; and $-N^+Me_3$. More preferably, $R^{6a}$ is a $C_1$ to $C_2$ alkyl group which is unsubstituted or substituted with a group selected from OH; $-NMe_2$; and $-N^+Me_3$.

Thus, $R^6$ is preferably $C_2$ to $C_4$ alkoxy which is unsubstituted or is substituted with a group selected from -OH; $-NR^{10}R^{11}$; $-N^+R^{10}R^{11}R^{12}$; and $-OR^{6a}$, wherein $R^{6a}$ is a $C_1$ to $C_3$ alkyl group which is unsubstituted or substituted with a group selected from OH; $-NR^{10}R^{11}$; and $-N^+R^{10}R^{11}R^{12}$. More preferably, $R^6$ is $C_2$ to $C_4$ alkoxy which is unsubstituted or is substituted with a group selected from -OH; $-NMe_2$; $-N^+Me_3$; and $-OR^{6a}$, wherein $R^{6a}$ is a $C_1$ to $C_3$ alkyl group which is unsubstituted or substituted with a group selected from $-NMe_2$; and $-N^+(Me)_3$. Preferably, $R^6$ is $C_2$ to $C_4$ alkoxy which is unsubstituted or is substituted with a group selected from -OH; $-NMe_2$; $-N^+(Me)_3$; and $-OR^{6a}$, wherein $R^{6a}$ is a $C_1$ to $C_2$ alkyl group which is substituted with a group selected from $-NMe_2$; and $-N^+(Me)_3$. Most preferrably, $R^6$ is $C_2$ to $C_4$ alkoxy which is unsubstituted or is substituted with a group selected from -OH; $-NMe_2$; $- N^+(Me)_3$; $-O(CH_2)-NMe_2$; and $-O(CH_2)-N^+(Me)_3$.

Typically, $R^6$ is bonded at the ring position marked as 1 below. If a group $R^5$ is present, this is typically present at the position marked as 2 below.

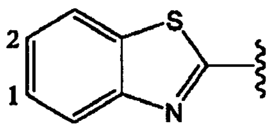

Thus, the indane of Formula (I) is typically an indane of Formula (IA):

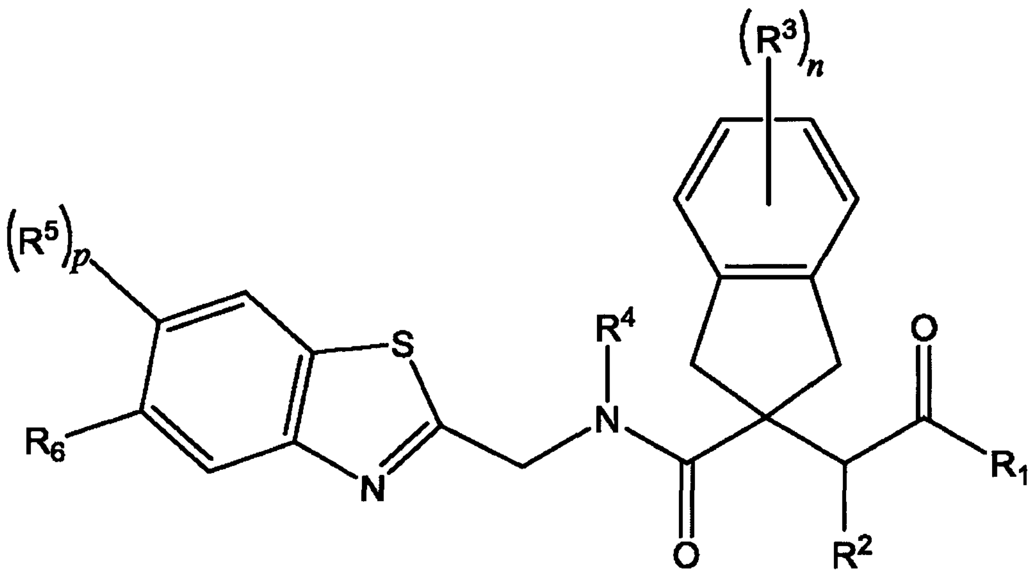

[FORMULA (IA)]

wherein $R^1$, $R^2$, $R^3$, $R^4$, $R^5$, $R^6$, *n* and *p* are as defined above.

Accordingly, preferred compounds of the invention are indanes of Formula (I) or Formula (IA) and pharmaceutically acceptable salts thereof wherein:

- $R^1$ is selected from OH, NHOH and $OR^{1a}$, or where the compound of Formula (I) contains a positively charged nitrogen atom, $R^1$ may be $O^-$, such that a $COO^-$ group is present and the compound forms a zwitterion;
- $R^2$ is selected from H and methyl;
- each $R^3$ group is independently selected from halogen (e.g. fluorine or chlorine) and -OH;
- *n* is an integer from 0 to 2;
- $R^4$ is H;

- $R^6$ is $C_2$ to $C_4$ alkoxy which is unsubstituted or is substituted with a group selected from -OH; $-NR^{10}R^{11}$; $-N^+R^{10}R^{11}R^{12}$; and $-OR^{6a}$, wherein $R^{6a}$ is a $C_1$ to $C_3$ alkyl group which is unsubstituted or substituted with a group selected from OH; $-NR^{10}R^{11}$; and $-N^+R^{10}R^{11}R^{12}$;
- *p* is 0 or 1;
- $R^5$ is methoxy, and
- $R^{10}$, $R^{11}$ and $R^{12}$ are independently H or methyl;
- with the proviso that the indane of Formula (I) is other than:

2-(2-(((4-ethoxybenzo[d]thiazol-2-yl)methyl)carbamoyl)-2,3-dihydro-1H-inden-2-yl)acetic acid;
2-[2-[(6-ethoxy-1,3-benzothiazol-2-yl)methylcarbamoyl]indan-2-yl]acetic acid;
2-[2-[[6-(2-hydroxyethoxy)-1,3-benzothiazol-2-yl]methylcarbamoyl]indan-2-yl]acetic acid;
2-[2-[[6-[2-(dimethylamino)ethoxy]-1,3-benzothiazol-2-yl]methylcarbamoyl]indan-2-yl]acetic acid;
2-[2-[[6-[2-(trimethylammonio)ethoxy]-1,3-benzothiazol-2-yl]methylcarbamoyl]indan-2-yl]acetate;
2-[2-[[5-[2-(dimethylamino)ethoxy]-1,3-benzothiazol-2-yl]methylcarbamoyl]indan-2-yl]acetic acid;
2-[2-[[5-[2-(trimethylammonio)ethoxy]-1,3-benzothiazol-2-yl]methylcarbamoyl]indan-2-yl]acetate;
2-(2-(((5-(3-(dimethylamino)propoxy)-6-methoxybenzo[d]thiazol-2-yl)methyl)carbamoyl)-5,6-difluoro-2,3-dihydro-1H-inden-2-yl)acetic acid;
2-(5,6-difluoro-2-(((6-methoxy-5-(3-(trimethylammonio)propoxy)benzo[d]thiazol-2-yl)methyl)carbamoyl)-2,3-dihydro-1H-inden-2-yl)acetate; and
2-(2-(((5-(2-(dimethylamino)ethoxy)-6-methoxybenzo[d]thiazol-2-yl)methyl)carbamoyl)-2,3-dihydro-1H-inden-2-yl)acetic acid.

More preferred compounds of the invention are indanes of Formula (I) or Formula (IA) and pharmaceutically acceptable salts thereof wherein:

- $R^1$ is OH, or where the compound of Formula (I) contains a positively charged nitrogen atom, $R^1$ may be $O^-$, such that a $COO^-$ group is present and the compound forms a zwitterion;
- $R^2$ is H;
- each $R^3$ group is independently selected from halogen, preferably fluorine;
- *n* is an integer from 0 to 2;
- $R^4$ is H;
- $R^6$ is $C_2$ to $C_4$ alkoxy which is unsubstituted or is substituted with a group selected from -OH; $-NMe_2$; $-N^+Me_3$; and $-OR^{6a}$, wherein $R^{6a}$ is a $C_1$ to $C_3$ alkyl group which is unsubstituted or substituted with a group selected from $-NMe_2$; and $-N^+(Me)_3$; most preferably $R^6$ is $C_2$ to $C_4$ alkoxy which is unsubstituted or substituted with a group selected from -OH; $-NMe_2$; $-N^+(Me)_3$; $-O(CH_2)-NMe_2$; and $-O(CH_2)-N^+(Me)_3$;
- *p* is 0 or 1;
- $R^5$ is methoxy; and
- $R^{10}$, $R^{11}$ and $R^{12}$ are independently H or methyl;
- with the proviso that the indane of Formula (I) is other than:

2-(2-(((4-ethoxybenzo[d]thiazol-2-yl)methyl)carbamoyl)-2,3-dihydro-1H-inden-2-yl)acetic acid;
2-[2-[(6-ethoxy-1,3-benzothiazol-2-yl)methylcarbamoyl]indan-2-yl]acetic acid;
2- [2- [[6-(2-hydroxyethoxy)-1,3-benzothiazol-2-yl]methylcarbamoyl]indan-2-yl]acetic acid;
2-[2-[[6-[2-(dimethylamino)ethoxy]-1,3-benzothiazol-2-yl]methylcarbamoyl]indan-2-yl]acetic acid;
2-[2-[[6-[2-(trimethylammonio)ethoxy]-1,3-benzothiazol-2-yl]methylcarbamoyl]indan-2-yl]acetate;
2-[2-[[5-[2-(dimethylamino)ethoxy]-1,3-benzothiazol-2-yl]methylcarbamoyl]indan-2-yl]acetic acid;
2-[2-[[5-[2-(trimethylammonio)ethoxy]-1,3-benzothiazol-2-yl]methylcarbamoyl]indan-2-yl]acetate;
2-(2-(((5-(3-(dimethylamino)propoxy)-6-methoxybenzo[d]thiazol-2-yl)methyl)carbamoyl)-5,6-difluoro-2,3-dihydro-1H-inden-2-yl)acetic acid;
2-(5,6-difluoro-2-(((6-methoxy-5-(3-(trimethylammonio)propoxy)benzo[d]thiazol-2-yl)methyl)carbamoyl)-2,3-dihydro-1H-inden-2-yl)acetate; and
2-(2-(((5-(2-(dimethylamino)ethoxy)-6-methoxybenzo[d]thiazol-2-yl)methyl)carbamoyl)-2,3-dihydro-1H-inden-2-yl)acetic acid.

Further preferred compounds of the invention are indanes of Formula (I) or (IA) and pharmaceutically acceptable salts thereof, wherein

- $R^1$, $R^2$, $R^3$, $R^4$, $R^5$ and *n* are as defined above;

- *p* is 1; and
- $R^6$ is $C_2$ to $C_4$ alkoxy which is substituted with a group selected from $-NR^{10}R^{11}$; $-N^+R^{10}R^{11}R^{12}$; and $-OR^{6a}$, wherein $R^{6a}$ is a $C_1$ to $C_3$ alkyl group which is unsubstituted or substituted with a group selected from $-NR^{10}R^{11}$; and $-N^+R^{10}R^{11}R^{12}$; and
- with the proviso that the indane of Formula (I) is other than:

  2-(2-(((5-(3-(dimethylamino)propoxy)-6-methoxybenzo[d]thiazol-2-yl)methyl)carbamoyl)-5,6-difluoro-2,3-dihydro-1H-inden-2-yl)acetic acid;
  2-(5,6-difluoro-2-(((6-methoxy-5-(3-(trimethylammonio)propoxy)benzo[d]thiazol-2-yl)methyl)carbamoyl)-2,3-dihydro-1H-inden-2-yl)acetate; and
  2-(2-(((5-(2-(dimethylamino)ethoxy)-6-methoxybenzo[d]thiazol-2-yl)methyl)carbamoyl)-2,3-dihydro-1H-inden-2-yl)acetic acid.

In this embodiment, $R^6$ is preferably $C_2$ to $C_4$ alkoxy which is substituted with a group selected from $-NMe_2$; $-N^+Me_3$; and $-OR^{6a}$, wherein $R^{6a}$ is a $C_1$ to $C_3$ alkyl group which is unsubstituted or substituted with a group selected from $-NMe_2$; and $-N^+(Me)_3$. Preferably, $R^6$ is $C_2$ to $C_4$ alkoxy which is substituted with a group selected from $-NMe_2$; $-N^+(Me)_3$; and - $OR^{6a}$, wherein $R^{6a}$ is a $C_1$ to $C_2$ alkyl group which is substituted with a group selected from - $NMe_2$; and $-N^+(Me)_3$. Most preferrably, $R^6$ is $C_2$ to $C_4$ alkoxy which is substituted with a group selected from $-NMe_2$; $-N^+(Me)_3$; $-O(CH_2)-NMe_2$; and $-O(CH_2)-N^+(Me)_3$.

Further preferred compounds of the invention are indanes of Formula (I) or (IA) and pharmaceutically acceptable salts thereof, wherein

- $R^1$, $R^2$, $R^3$, $R^4$, $R^5$, *n* and *p* are as defined above; and
- $R^6$ is $C_2$ to $C_4$ alkoxy which is substituted with a group selected from $-OR^{6a}$ and $-NR^{10}R^{6a}$, wherein $R^{6a}$ is a $C_1$ to $C_3$ alkyl group which is unsubstituted or substituted with a group selected from OH; $-NR^{10}R^{11}$; $-N^+R^{10}R^{11}R^{12}$; $-NR^{10}NR^{11}R^{12}$; $-NR^{10}N^+R^{11}R^{12}R^{13}$; $-N^+R^{10}R^{11}NR^{12}R^{13}$; $-NR^{10}C(NR^{11})NR^{12}R^{13}$; $-NR^{10}C(N^+R^{11}R^{12})NR^{13}R^{14}$; $-C(NR^{10})NR^{11}R^{12}$; and $-C(N^+R^{10}R^{11})NR^{12}R^{13}$.

Preferably in this embodiment $R^6$ is $C_2$ to $C_4$ alkoxy which is substituted with a group $-OR^{6a}$, wherein $R^{6a}$ is a $C_1$ to $C_3$ alkyl group which is unsubstituted or substituted with a group selected from OH; $-NR^{10}R^{11}$; and $-N^+R^{10}R^{11}R^{12}$. More preferably, $R^{6a}$ is a $C_1$ to $C_3$ alkyl group which is unsubstituted or substituted with a group selected from $-NMe_2$; and - $N^+(Me)_3$. More preferably, $R^{6a}$ is a $C_1$ to $C_2$ alkyl group which is substituted with a group selected from $-NMe_2$; and $-N^+(Me)_3$. Most preferrably, $R^{6a}$ is $-O(CH_2)-NMe_2$; or $-O(CH_2)-N^+(Me)_3$.

Preferred compounds of the invention are:

2-[2-[[6-(3-hydroxypropoxy)-1,3-benzothiazol-2-yl]methylcarbamoyl]indan-2-yl]acetic acid;
2-[2-[(6-propoxy-1,3-benzothiazol-2-yl)methylcarbamoyl]indan-2-yl]acetic acid;
2-[2-[[5-[3-(dimethylamino)propoxy]-6-methoxy-1,3-benzothiazol-2-yl]methylcarbamoyl]indan-2-yl] acetic acid;
2-[2-[[6-methoxy-5-[3-(trimethylammonio)propoxy]-1,3-benzothiazol-2-yl]methylcarbamoyl] indan-2-yl] acetate;
2-[2-[[6-methoxy-5-[2-[2-(trimethylammonio)ethoxy]ethoxy]-1,3-benzothiazol-2-yl]methylcarbamoyl] indan-2-yl] acetate;
2-[5,6-difluoro-2-[[6-methoxy-5-[2-[2-(trimethylammonio)ethoxy]ethoxy]-1,3-benzothiazol-2-yl]methylcarbamoyl]indan-2-yl]acetate;
2-[5,6-difluoro-2-[[6-methoxy-5-[2-(trimethylammonio)ethoxy]-1,3-benzothiazol-2-yl]methylcarbamoyl]indan-2-yl]acetate;
2-(2-(((5-(4-(dimethylamino)butoxy)-6-methoxybenzo[d]thiazol-2-yl)methyl)carbamoyl)-2,3-dihydro-1H-inden-2-yl)acetic acid;
2-(2-(((6-methoxy-5-(4-(trimethylammonio)butoxy)benzo[d]thiazol-2-yl)methyl)carbamoyl)-2,3-dihydro-1H-inden-2-yl)acetate; and
and pharmaceutically acceptable salts thereof.

Particularly preferred compounds of the invention are:

2-[2-[[6-(3-hydroxypropoxy)-1,3-benzothiazol-2-yl]methylcarbamoyl]indan-2-yl] acetic acid;
2-[2-[(6-propoxy-1,3-benzothiazol-2-yl)methylcarbamoyl]indan-2-yl]acetic acid;
2-[2-[[5-[3-(dimethylamino)propoxy]-6-methoxy-1,3-benzothiazol-2-yl]methylcarbamoyl]indan-2-yl] acetic acid;
2-[2-[[6-methoxy-5-[3-(trimethylammonio)propoxy]-1,3-benzothiazol-2-yl]methylcarbamoyl]indan-2-yl]acetate;
2-[2-[[6-methoxy-5-[2-[2-(trimethylammonio)ethoxy]ethoxy]-1,3-benzothiazol-2-yl]methylcarbamoyl]indan-2-yl] acetate;
2-[5,6-difluoro-2-[[6-methoxy-5-[2-[2-(trimethylammonio)ethoxy]ethoxy]-1,3-benzothiazol-2-yl]methylcarbamoyl]indan-2-yl]acetate;
2-[5,6-difluoro-2-[[6-methoxy-5-[2-(trimethylammonio)ethoxy]-1,3-benzothiazol-2-yl]methylcarbamoyl]indan-2-yl]acetate; and
and pharmaceutically acceptable salts thereof.

Most preferred compounds are 2-[5,6-difluoro-2-[[6-methoxy-5-[2-[2-(trimethylammonio)ethoxy]ethoxy]-1,3-benzothiazol-2-yl]methylcarbamoyl]indan-2-yl]acetate; 2-[2-[[6-methoxy-5-[3-(trimethylammonio)propoxy]-1,3-benzothiazol-2-yl]methylcarbamoyl]indan-2-yl]acetate; 2-[2-[[6-methoxy-5-[2-[2-(trimethylammonio)ethoxy]ethoxy]-1,3-benzothiazol-2-yl]methylcarbamoyl]indan-2-yl]acetate and pharmaceutically acceptable salts of these compounds.

*Synthesis*

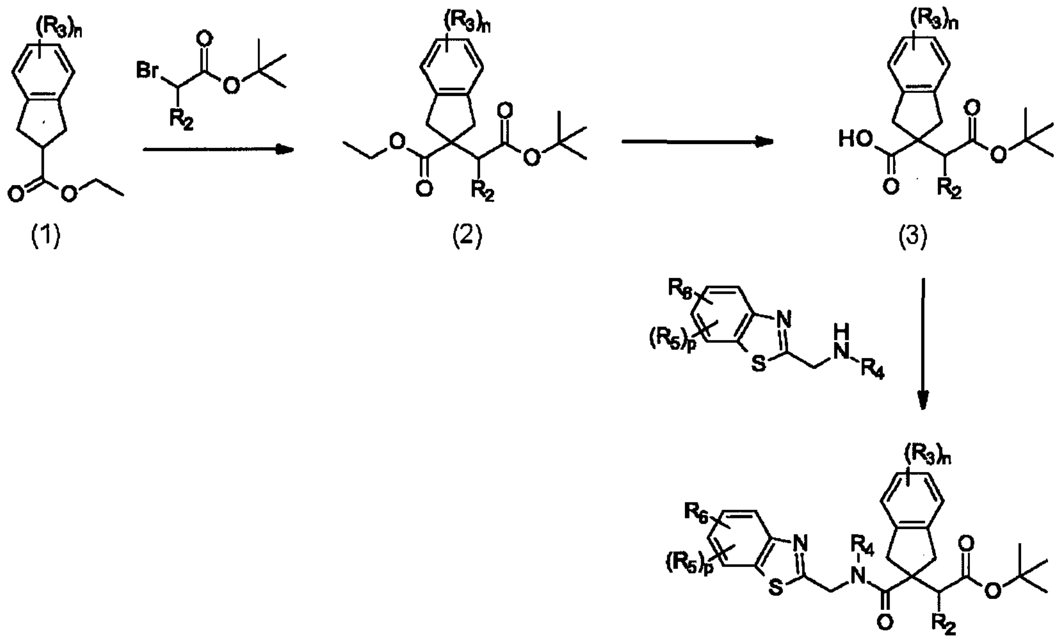

The compounds of the invention can be prepared by any suitable method. For example, as described in more detail below, deprotonation of commercially available ethyl esters (1) with strong base (such as sodium hexamethyldisilazide) then alkylation of the anion with *tert-butyl* bromoacetates gives diester (2) (Bell, I.M. and Stump, C.A., WO2006/29153; Robinson, R.P. et al, Bioorganic and Medicinal Chemistry Letters, 1996, 1719). Basic hydrolysis of the ethyl ester in the presence of the *tert-butyl* ester gives (3). Amide formation with a suitable 2-aminomethyl benzothiazole followed by treatment with TFA to remove the *tert-butyl* ester then affords the desired acids. Examples of suitable protocols for formation of amino-methyl benzothiazoles are provided below. The acids can be converted to esters ($R^1 = OR^{1a}$) or other produrug forms ($R^1 = OCH_2OC(O)R^{1a}$) by techniques known to the skilled person.

There are numerous ways of accessing hydroxamic acids (for a review see Ganeshpurkar, A., et al, Current Organic Syntheses, 2018, 15, 154-165) but a very reliable procedure is to couple acids with O-(oxan-2-yl)hydroxylamine using peptide coupling conditions to give protected hydroxamates then deprotect with TFA to generate the hydroxamic acids (see for example Ding, C., et al, Bioorg. Med. Chem. Lett, 2017, 25, 27-37).

*Compositions and Combinations*

The present invention also provides a pharmaceutical composition, the pharmaceutical composition comprising a compound of the invention together with a pharmaceutically acceptable carrier or diluent. Typically, the composition contains up to 85 wt% of a compound of the invention. More typically, it contains up to 50 wt% of a compound of the invention. Preferred pharmaceutical compositions are sterile and pyrogen free. Further, when the pharmaceutical compositions provided by the invention contain a compound of the invention which is optically active, the compound of the

invention is typically a substantially pure optical isomer.

The composition of the invention may be provided as a kit comprising instructions to enable the kit to be used in the methods described herein or details regarding which subjects the method may be used for.

As explained above, the compounds of the invention are useful in treating or preventing bacterial infection. In particular, they are useful as inhibitors of LasB, in particular LasB of *Pseudomonas aeruginosa* (PA). The compounds may be used alone or they may be used in combination therapies with antibiotic agents, to enhance the action of the antibiotic agent. The present invention therefore also provides a combination comprising (i) a compound of the invention as described herein and (ii) an antibiotic agent. The combination may further comprise one or more additional active agents. The compound of the invention and the antibiotic agent may be provided in a single formulation, or they may be separately formulated. Where separately formulated, the two agents may be administered simultaneously or separately. They may be provided in the form of a kit, optionally together with instructions for their administration.

Where formulated together, the two active agents may be provided as a pharmaceutical composition comprising (i) a compound of the invention as described herein and (ii) a further antibacterial compound; and (iii) a pharmaceutically acceptable carrier or diluent.

Preferably, the antibiotic agent is efficacious against *Pseudomonas* infection. Most preferably, the antibiotic is tobramycin, neomycin, streptomycin, gentamycin, ceftazidime, ticarcillin, piperacillin, tazobactam, imipenem, meropenem, rifampicin, ciprofloxacin, amikacin, colistin, aztreonam or levofloxacin.

The compound or combination of the invention may be administered in a variety of dosage forms. Thus, they can be administered orally, for example as tablets, troches, lozenges, aqueous or oily suspensions, dispersible powders or granules. They may also be administered parenterally, whether subcutaneously, intravenously, intramuscularly, intrasternally, transdermally or by infusion techniques. The compound or combination may also be administered as a suppository. Preferably, the compound or combination may be administered via inhaled (aerosolised) or intravenous administration, most preferably by inhaled (aerosolised) administration.

The compound or combination of the invention is typically formulated for administration with a pharmaceutically acceptable carrier or diluent. For example, solid oral forms may contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols; binding agents; e.g. starches, arabic gums, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates, laurylsulphates; and, in general, non toxic and pharmacologically inactive substances used in pharmaceutical formulations. Such pharmaceutical preparations may be manufactured in known manner, for example, by means of mixing, granulating, tableting, sugar coating, or film coating processes.

The compound or combination of the invention may be formulated for inhaled (aerosolised) administration as a solution or suspension. The compound or combination of the invention may be administered by a metered dose inhaler (MDI) or a nebulizer such as an electronic or jet nebulizer. Alternatively, the compound or combination of the invention may be formulated for inhaled administration as a powdered drug, such formulations may be administered from a dry powder inhaler (DPI). When formulated for inhaled administration, the compound or combination of the invention may be delivered in the form of particles which have a mass median aerodynamic diameter (MMAD) of from 1 to 100 $\mu$m, preferably from 1 to 50 $\mu$m, more preferably from 1 to 20 $\mu$m such as from 3 to 10 $\mu$m, e.g. from 4 to 6 $\mu$m. When the compound or combination of the invention is delivered as a nebulized aerosol, the reference to particle diameters defines the MMAD of the droplets of the aerosol. The MMAD can be measured by any suitable technique such as laser diffraction.

Liquid dispersions for oral administration may be syrups, emulsions and suspensions. The syrups may contain as carriers, for example, saccharose or saccharose with glycerine and/or mannitol and/or sorbitol.

Suspensions and emulsions may contain as carrier, for example a natural gum, agar, sodium alginate, pectin, methylcellulose, carboxymethylcellulose, or polyvinyl alcohol. The suspension or solutions for intramuscular injections or inhalation may contain, together with the active compound, a pharmaceutically acceptable carrier, e.g. sterile water, olive oil, ethyl oleate, glycols, e.g. propylene glycol, and if desired, a suitable amount of lidocaine hydrochloride.

Solutions for inhalation, injection or infusion may contain as carrier, for example, sterile water or preferably they may be in the form of sterile, aqueous, isotonic saline solutions. Pharmaceutical compositions suitable for delivery by needleless injection, for example, transdermally, may also be used.

*Therapeutic Efficacy*

The compounds, compositions and combinations of the present invention are therapeutically useful. The present invention therefore provides compounds, compositions and combinations as described herein, for use in medicine. The present invention provides compounds as described herein, for use in treating the human or animal body. For the avoidance of doubt, the agent may comprise a compound of the invention in the form of a solvate.

The compounds, compositions and combinations of the invention are useful in treating or preventing bacterial

infection. The present invention therefore provides a compound, combination or composition as described herein for use in a method of treating or preventing bacterial infection in a subject in need thereof. Also provided is a method for treating or preventing bacterial infection in a subject in need thereof, which method comprises administering to said subject an effective amount of a compound, combination or composition as described herein. Further provided is the use of a compound, combination or composition as described herein in the manufacture of a medicament for use in treating or preventing bacterial infection in a subject.

The compounds described herein are useful as inhibitors of LasB, in particular LasB of *Pseudomonas aeruginosa* (PA). The inhibition of LasB in the bacteria prevents LasB secreted by bacteria from hydrolysing host tissue and host immune-response proteins, thereby supporting the subject in its natural response to bacterial infection and inflammation. The compounds described herein are therefore useful as standalone adjuncts in antibacterial therapy, for example in chemotherapy regimes. Further, the compounds are useful in inhibiting biofilm formation, and/or in disrupting a biofilm. This activity in preventing biofilm formation or disrupting established biofilms facilitates antibiotic agents in eradication of bacterial infection. It also facilitates the host's own immune system in attacking the bacterial infection. The compounds may therefore be used as stand alone antibacterial agents.

Alternatively, the compounds described herein may be used in combination with antibiotic agents to enhance the action of the antibiotic agent. Therefore, further provided is a compound of the invention as described herein for use in a method of treating or preventing bacterial infection by co-administration with an antibiotic agent. Also provided is a method for treating or preventing bacterial infection in a subject in need thereof, which method comprises co-administering to said subject an effective amount of a compound as described herein and an antibiotic agent. Also provided is the use of a compound as described herein in the the manufacture of a medicament for use in treating or preventing bacterial infection by co-administration with an antibiotic agent.

In one aspect, the subject is a mammal, in particular a human. However, it may be non-human. Preferred non-human animals include, but are not limited to, primates, such as marmosets or monkeys, commercially farmed animals, such as horses, cows, sheep or pigs, and pets, such as dogs, cats, mice, rats, guinea pigs, ferrets, gerbils or hamsters. The subject can be any animal that is capable of being infected by a bacterium.

The compounds, compositions and combinations described herein are useful in the treatment of bacterial infection which occurs after a relapse following an antibiotic treatment. The compounds and combinations can therefore be used in the treatment of a patient who has previously received antibiotic treatment for the (same episode of) bacterial infection.

The bacterium causing the infection may be any bacterium expressing LasB or an analogue thereof. Typically the bacterium causing the infection expresses LasB. The bacterium may, for instance, be any bacterium that can form a biofilm. The bacterium may be Gram-positive or Gram-negative. In a preferred instance the bacterium is Gram-negative. The bacterium may in particular be a pathogenic bacterium.

The bacterial infection may be caused by *Bacillus, Pseudomonas, Staphylococcus, Streptococcus, Listeria, Escherichia* or *Burkholderia.* For example, the bacterium may be one selected from *Staphylococcus aureus, Haemophilus influenza, Pseudomonas aeruginosa* and *Burkholderia cepacia.*

In one preferred instance, the bacterium may be one selected from a bacterium of the family Pseudomonadaceae. For example, the bacterium may be selected from one of the following genera: *Pseudomonas, Azomonas, Azomonotrichon, Azorhizophilus, Azotobacter, Cellvibrio, Mesophilobacter, Rhizobacter, Rugamonas* and *Serpens.* Preferably the bacterium is a *Pseudomonas,* particularly where the condition to be treated is pneumonia. The bacterium may be an opportunistic pathogen. The bacterium may be selected from *Pseudomonas aeruginosa, Pseudomonas oryzihabitans,* and *Pseudomonas plecoglossicida,* and most preferably, the bacterium is *Pseudomonas aeruginosa* (PA).

The compound, composition or combination of the invention may be used to treat or prevent infections and conditions caused by any one or a combination of the above-mentioned bacteria. In particular, the compound or combination of the invention may be used in the treatment or prevention of pneumonia. The compound or combination may also be used in the treatment of septic shock, urinary tract infection, and infections of the gastrointestinal tract, skin or soft tissue.

The compounds and combinations are particularly useful in the treatment of patients suffering from cystic fibrosis. Preferably, the compound or combination of the invention may be used in the treatment or prevention of pneumonia in a subject suffering from cystic fibrosis. For example, the subject may have any of the six CFTR mutation classes, and/or may be infected by or chronically colonised by PA. The compounds and combinations of the invention may also be used in the treatment of neutropenic patients.

A compound or combination of the invention can be administered to the subject in order to prevent the onset or reoccurrence of one or more symptoms of the bacterial infection. This is prophylaxis. In this embodiment, the subject can be asymptomatic. The subject is typically one that has been exposed to the bacterium. A prophylactically effective amount of the agent or formulation is administered to such a subject. A prophylactically effective amount is an amount which prevents the onset of one or more symptoms of the bacterial infection.

A compound or combination of the invention can be administered to the subject in order to treat one or more

symptoms of the bacterial infection. In this embodiment, the subject is typically symptomatic. A therapeutically effective amount of the agent or formulation is administered to such a subject. A therapeutically effective amount is an amount effective to ameliorate one or more symptoms of the disorder.

A therapeutically or prophylactically effective amount of the compound of the invention is administered to a subject. The dose may be determined according to various parameters, especially according to the compound used; the age, weight and condition of the subject to be treated; the route of administration; and the required regimen. Again, a physician will be able to determine the required route of administration and dosage for any particular subject. A typical daily dose is from about 0.01 to 100 mg per kg, preferably from about 0.1 mg/kg to 50 mg/kg, e.g. from about 1 to 10 mg/kg of body weight, according to the activity of the specific inhibitor, the age, weight and conditions of the subject to be treated, the type and severity of the disease and the frequency and route of administration. Preferably, daily dosage levels are from 5 mg to 2 g.

*Other Uses*

The antibacterial properties of the compounds described herein mean that they are also useful in the treatment of bacterial infection *in vitro,* i.e. other than by the treatment of human or animal subjects. Thus, also described herein is a cleaning composition comprising a indane derivative of Formula (I) or a salt thereof. The cleaning composition may further comprise, for example, a detergent, a surfactant (including ionic and non-ionic surfactants), a diluent, a bleach (including a hypochlorite such as sodium hypochlorite or calcium hypochlorite, chlorine, chlorine dioxide, hydrogen peroxide or an adduct thereof, sodium perborate, and sodium percarbonate), an alcohol (such as ethanol or isopropanol), or a disinfectant. Typically, the disinfectant may be selected from benzyl-4-chlorophenol, amylphenol, phenylphenol, glutaraldehyde, alkyl dimethyl benzyl ammonium chloride, alkyl dimethyl ethylbenzyl ammonium chloride, iodine, peracetic acid and chlorine dioxide. Typically, the detergent may be an alkaline detergent such as sodium hydroxide, sodium metasilicate, or sodium carbonate, or an acid detergent such as hydrochloric acid, nitric acid, sulfuric acid, phosphoric acid, citric acid, or tartaric acid.

Also described herein is the use of the indane derivative of Formula (I) as described herein for the prevention or treatment of bacterial contamination *in vitro.* Such use may be an *in vitro* method for the prevention or treatment of bacterial infection which comprises a step of treatment of an object with a compound or combination of the invention. Such use is a non-therapeutic use and may involve, for example, prevention or treatment of bacterial contamination on a surface, such as a surface of an indwelling medical device, or an object used in a clinical setting. The surface may be the surface of a catheter, a nebulizer, a ventilator, or a face mask. Typically, the bacterial contamination is caused by any bacteria described herein. Preferably, the bacteria is *Pseudomonas aeruginosa.*

The following Examples illustrate the invention. They do not however, limit the invention in any way. In this regard, it is important to understand that the particular assay used in the Examples section is designed only to provide an indication of biological activity. There are many assays available to determine biological activity, and a negative result in any one particular assay is therefore not determinative.

**Experimental Details**

*General synthetic methodology*

As described below, there are two synthetic methodologies to synthesize the compounds of the invention.

Method A. Regiospecific synthesis of key intermediate (3)

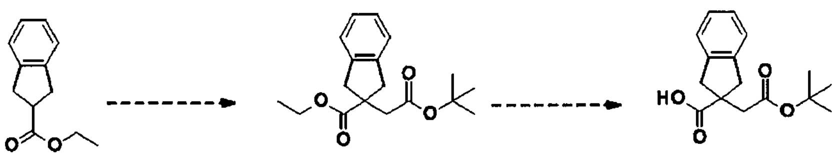

(1) (2) (3)

Scheme 1

Deprotonation of commercially available ethyl ester (1) with strong base (such as sodium hexamethyldisilazide) then alkylation of the anion with tert-butyl bromoacetate gives known diester (2) (Bell, I.M. and Stump, C.A.,

WO2006/29153; Robinson, R.P. et al, Bioorganic and Medicinal Chemistry Letters, 1996, 1719). Basic hydrolysis of the ethyl ester in the presence of the *tert*-butyl ester gives (3). Amide formation with a suitable 2-aminomethyl benzothiazole followed by treatment with TFA to remove the *tert-butyl* ester then affords the desired acids. The acids can be converted to esters ($R^1 = R^{1a}$) or other produrug forms ($R^1 = CH_2OC(O)R^{1a}$) by techniques known to the skilled person.

This methodology can be adapted to substituents on the indane ring.

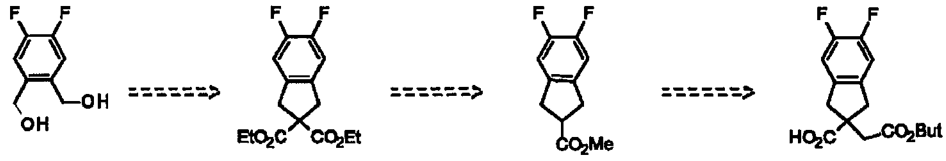

(4) (5) (6) (7)

Scheme 2

For example commercially available diol [4,5-difluoro-2-(hydroxymethyl)phenyl]methanol (4) can be converted into the bis bromomethyl analogue with either HBr (WO2008/151211) or phosphorus tribromide (US2006/223830) which can further be reacted with diethyl malonate to give indane (5), (Scheme 2). Standard hydrolysis of both esters followed by mono decarboxylation affords the mono acid (WO2006/125511) which can be esterified to give (6), the difluoro analogue of (1). Using the same methodology as applied to (1) then affords key acid (7), the difluoro analogue of intermediate (3). Similar chemistry can be applied to the corresponding analogues having different substituents on the indane ring.

There are numerous ways of accessing hydroxamic acids (for a review see Ganeshpurkar, A., et al, Current Organic Syntheses, 2018, 15, 154-165) but a very reliable procedure is to couple acids (64) with O-(oxan-2-yl)hydroxylamine using peptide coupling conditions to give protected hydroxamates (65) then deprotect with TFA to generate the hydroxamic acids (66), (see for example Ding, C., et al, Bioorg. Med. Chem. Lett, 2017, 25, 27-37).

Method B. Synthesis of protected 2-aminomethyl benzothiazoles

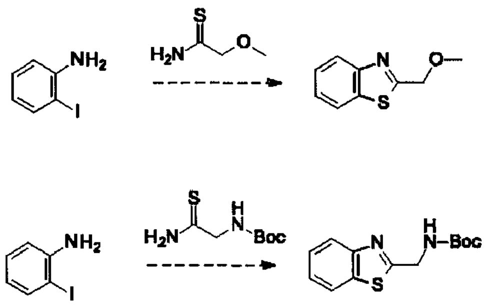

Eq. 1

Eq. 2

(8)

Scheme 3

There are many ways of constructing benzothiazoles (for a review, see Seth, S; "A Comprehensive Review on Recent advances in Synthesis & Pharmacotherapeutic potential of Benzothiazoles", Anti-Inflammatory & Anti-Allergy Agents in Medicinal Chemistry, 2015, 14, 98-112). However, most methods afford alkyl substitution at the C2-position necessitating further functional group manipulation to access the desired aminomethyl substituent required in this invention. In the 1980's the pioneering work of Takagi and colleagues led to a palladium-catalysed method of directly producing functionalised methyl groups (see Eq. 1, Scheme 3; Takagi, K. et al, Chemistry Letters, 1987, 16, 839-840). This chemistry was recently rediscovered by Mutabilis scientists who adapted the methodology to introduce a protected aminomethyl group into the benzothiazole core (8), (see Eq. 2, Scheme 3; Desroy, N., et al, Journal of Medicinal Chemistry, 2013, 56, 1418-1430). Application of this methodology accesses the protected 2-aminomethyl benzothiazoles of this invention.

Method C. Functional group manipulation on protected aminomethylbenzothiazole

In many cases the desired substituent pattern on the phenyl ring can be established prior to benzothiazole

formation using standard functional group transformations. In certain cases it is preferred to perform functional group transformations after benzothiazole formation.

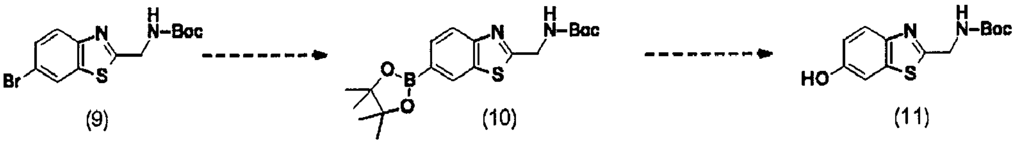

(9) (10) (11)

Scheme 4

For instance, in order to access a phenolic intermediate on the benzothiazole, one method (Scheme 4) is to construct the benzothiazole with a bromo substituent (9) then displace the bromide using bis(pinacolato)diboron and catalytic Pd(dppf)$Cl_2$.$CH_2Cl_2$, affording the boronate ester (10) (for a related example see Malinger, A. et al, Journal of Medicinal Chemistry, 2016, 59, 1078-1101). Oxidation of the boronic ester to the phenol (11) can be accomplished with hydrogen peroxide (see Liu, J. et al, Tetrahedron Letters, 2017, 58, 1470-1473.) Further derivatisation of the phenol group can be achieved by standard alkylation reactions familiar to those skilled in the art.

Method D. Final stages to synthesise the Examples

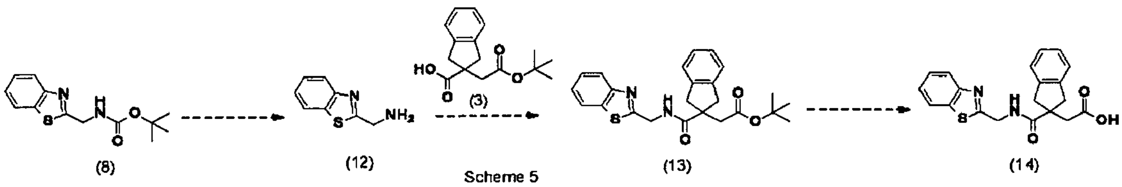

(8) (12) (3) (13) (14)

Scheme 5

The final stages of the syntheses generally involve acid-catalysed removal of the BOC group from (8) to reveal the free amines (12) followed by coupling with acids of type (3), usually with the standard peptide coupling reagent HATU (for a comprehensive review of the myriad available peptide coupling reagents, see Valeur, E. and Bradley, M, Chem. Soc. Rev., 2008, 28, 606-631). Finally further acid treatment with TFA removes the t-butyl ester to afford the Examples of the invention.

Method E. Functional group manipulation after amide coupling of aminomethylbenzothiazole and indanyl moieties

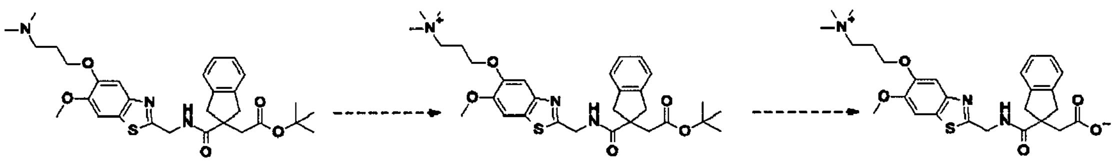

(15) (16) (17)

Scheme 6

As an example of this approach, alkylation of tert-butyl N-[(5-hydroxy-6-methoxy-1,3-benzothiazol-2-yl)methyl]carbamate with 3-chloro-N,N-dimethylpropan-1-amine, removal of the tert-butoxycarbonyl protecting group and coupling with acid (3) can generate the N,N-dimethylaminopropyloxy intermediate (15). Reaction with an alkylating agent such as iodomethane then generates the corresponding quaternary ammonium salt (16) and finally removal of the tert-butyl ester reveals the carboxylate acid, generating zwitterionic (17) containing both a positive and a negative charge.

It is understood that these synthetic routes are not exclusive and functional group interconversion is possible at the phenyl precursor stage, the protected aminomethyl benzothiazole stage and the post-coupling amide stage.

## Examples

1H NMR spectra are reported at 300, 400 or 500 MHz in DMSO-d6 solutions ($\delta$ in ppm), using DMSO-$d_5$ as

reference standard (2.50 ppm), or $CDCl_3$ solutions using chloroform as the reference standard (7.26 ppm). When peak multiplicities are reported, the following abbreviations are used: s (singlet), d (doublet), t (triplet), m (multiplet), bs (broadened singlet), bd (broadened doublet), dd (doublet of doublets), dt (doublet of triplets), q (quartet). Coupling constants, when given, are reported in hertz (Hz).

The term "purified by prep hplc (MDAP)" refers compound purification using a mass-directed auto purification system on an Agilent 1260 infinity machine with an XSelect CHS Prep C18 column, eluting with 0.1% FA in water/ACN and detection with a Quadrupole LC/MS.

*Abbreviations*

| | |
|---|---|
| ACN | Acetonitrile |
| AcOH | Acetic acid |
| aq. | Aqueous |
| Bpin | Bis(pinacolato)diboron |
| $CaCl_2$ | Calcium chloride |
| $Cs_2CO_3$ | Cesium carbonate |
| cfu | Colony forming unit |
| Conc | Concentrated |
| $Cu(OAc)_2$ | Copper(II) acetate |
| CuO | Copper oxide |
| DCM | Dichloromethane |
| DEA | Diethylamine |
| DIPEA | N,N-Diisopropylethylamine |
| DMAP | 4-Dimethylaminopyridine |
| DMF | N,N-Dimethylformamide |
| DMSO | Dimethyl sulfoxide |
| dppf | 1,1'-Bis(diphenylphosphino)ferrocene |
| EDC.HCl | N-(3-Dimethylaminopropyl)-N'-ethylcarbodiimide hydrochloride |
| $Et_2O$ | Diethyl ether |
| EtOAc | Ethyl acetate |
| EtOH | Ethanol |
| $Et_3N$ | Triethylamine |
| Ex | Excitation |
| FA | Formic acid |
| FCC | Flash column chromatography purification on silica |
| h | Hour(s) |
| HATU | 1-[Bis(dimethylamino)memylene]-1H-1,2,3-triazolo[4,5-b]pyridinium 3-oxid hexafluorophosphate |
| HCl | Hydrochloric acid/hydrochloride salt |
| HOBt | Hydroxybenzotriazole |
| H2SO4 | Sulfuric Acid |
| Km | Michaelis constant |
| KOAc | Potassium acetate |
| KOH | Potassium hydroxide |
| MeCN | Acetonitrile |
| MeI | Methyl iodide |
| MeOH | Methanol |
| min | Minute(s) |
| $MgSO_4$ | Magnesium sulfate |
| $N_2$ | Nitrogen |
| NBS | N-bromo succinimide |
| $Na_2CO_3$ | Sodium carbonate |
| $NaHCO_3$ | Sodium bicarbonate |
| NaHMDS | Sodium bis(trimethylsilyl)amide |
| $Na_2SO_4$ | Sodium sulfate |
| $Pd_2(dba)_3$ | Tris(dibenzylideneacetone)dipalladium(0) |
| $PdCl_2(dppf)$ | [1,1'-Bis(diphenylphosphino)ferrocene]dichloropalladium(II) |

| | |
|---|---|
| RT | Room temperature |
| SCX-2 | Strong cation exchange resin (silica-propyl sulfonic acid) |
| T%B | Time, % solvent B |
| TES | Triethylsilane |
| TFA | Trifluoroacetic acid |
| THF | Tetrahydrofuran |
| T3P | Propylphosphinic anhydride |

**Example 1 2-[2-[[6-(3-hydroxypropoxy)-1,3-benzothiazol-2-yl]methylcarbamoyl]indan-2-yl]acetic acid**

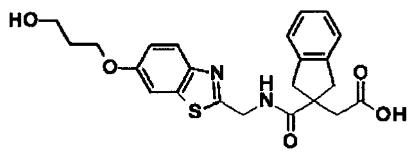

a. Tert-butyl N-[(6-bromo-1,3-benzothiazol-2-yl)methyl]carbamate

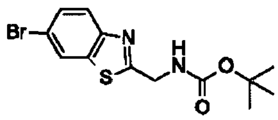

To a stirred solution of 4-bromo-2-iodo-aniline (3 g, 10.13 mmol) and tert-butyl (2-amino-2-thioxoethyl) carbamate (1.92 g, 10.13 mmol) in DMF (30 mL) was added CuO (0.8 g, 10.13 mmol) at room temperature and the reaction mixture was degassed with argon for 15 minutes. Then Dppf (280 mg, 0.50 mmol) and $Pd_2(dba)_3$ (185.4 mg, 0.20) were added and the resulting reaction mixture was degassed with argon for further 5 minutes. The reaction mixture was stirred in sealed tube at 60 °C for 3h, and then filtered through celite pad and washed the pad with EtOAc (50 mL). The filtrate was washed with water (2x30 mL) and concentrated under reduced pressure. The crude compound was purified by silica gel chromatography eluting with 22% EtOAc in petroleum ether affording as a yellow solid (5 g, 72%). M/z 343 $(M+H)^+$.

b. Tert-butyl N-[[6-(4,4,5,5-tetramethyl-1,3,2-dioxaborolan-2-yl)-1,3-benzothiazol-2-yl]methyl] carbamate

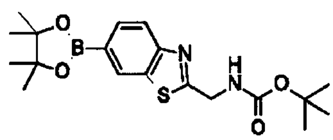

To a stirred solution of tert-butyl N-[(6-bromo-1,3-benzothiazol-2-yl)methyl]carbamate (1.3 g, 3.80 mmol), and bis(pinacolato)diboron (1.44 g, 5.70 mmol) in 1,4-dioxane (15 mL) was added KOAc (745 mg, 7.60 mmol) at room temperature and the reaction mixture was purged with argon for 15 minutes. Then $PdCl_2$(dppf).DCM (155 mg, 0.190 mmol) was added and the reaction mixture purged with argon for further 5 minutes. The reaction mixture was stirred to reflux in sealed tube for 12 h, and then filtered through celite pad and washed with EtOAc (50 mL). The filtrate was washed with water (2 x 30 mL), the organic layer was dried with sodium sulphate, filtered and concentrated under reduced pressure to get a brown solid (1.5 g, crude). M/z 391.2 $(M+H)^+$.

c. Tert-butyl N-[(6-hydroxy-1,3-benzothiazol-2-yl)methyl]carbamate

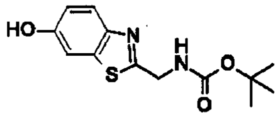

To a stirred solution of tert-butyl N-[[6-(4,4,5,5-tetramethyl-1,3,2-dioxaborolan-2-yl)-1,3-benzothiazol-2-yl]methyl]carbamate (1.5 g, 3.84 mmol) in THF (15 mL) was added IN NaOH (3.84 mL g, 3.84 mmol) at 0 °C and stirred for 10 minutes. Then $H_2O_2$ (30% in $H_2O$, 0.21 mL, 8.84 mmol) was added at 0 °C and the reaction mixture stirred to room temperature for 1 h. The reaction mixture was partitioned between ethyl acetate (100 mL) and water (70 mL). The aqueous phase was extracted with ethyl acetate (2 x 100 mL) and the combined organic extracts were washed with brine, dried with $Na_2SO_4$, filtered and evaporated. The crude product was purified by silica gel chromatography eluting with 40% EtOAc in petroleum ether affording a white solid (1.0 g, 93.4%). M/z 281.1 $(M+H)^+$.

d. Tert-butyl N-[[6-(3-hydroxypropoxy)-1,3-benzothiazol-2-yl]methyl]carbamate

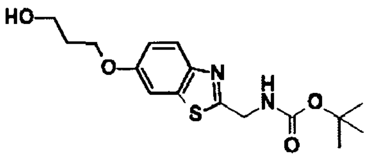

To a solution of tert-butyl N-[(6-hydroxy-1,3-benzothiazol-2-yl)methyl]carbamate (300 mg, 1.07 mmol) in DMF (5 mL) was added $K_2CO_3$ (222 mg, 1.60 mmol), 3-bromopropan-1-ol (224 mg, 1.60 mmol) at room temperature and heated at 80 °C for 2 h. The reaction mixture was diluted with water (20 mL) and extracted with EtOAc (2 x 30 mL). The combined organic extract was dried, filtered and evaporated. The crude was purified by silica gel chromatography eluting with 45-60% EtOAc in petroleum ether affording a yellow solid (210 m, 58%). M/z = 338.9 $(M+H)^+$.

e. 3-[[2-(aminomethyl)-1,3-benzothiazol-6-yl]oxy]propan-1-ol hydrochloride

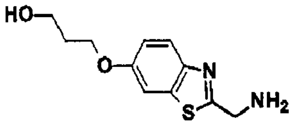

To a solution of tert-butyl N-[[6-(3-hydroxypropoxy)-1,3-benzothiazol-2-yl]methyl]carbamate (210 mg, 0.62 mmol) in dioxane (5 mL) was added 4M HCl in dioxane (2 mL) at room temperature and stirred for 3 h. The reaction mixture was evaporated and the resulting residue was triturated with diethyl ether (20 mL) affording an off white solid (165 mg, crude). M/z = 238.9 $(M+H)^+$.

f. Methyl indane-2-carboxylate

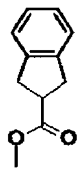

To a stirred solution of 2,3-dihydro-1H-indene-2-carboxylic acid (20 g, 123 mmol) in methanol (200 mL) was added conc. $H_2SO_4$ (10 mL, 185 mmol) drop wise at room temperature and stirred at 80 °C for 16 h. The reaction mixture was evaporated to get residue. The residue was dissolved in water (100 mL) and extracted with EtOAc (2x100 mL). The

organic layer was washed with sat. sodium bicarbonate, brine and evaporated affording a light brown liquid (20 g, 92%). M/z 177.1 $(M+H)^+$.

g. Methyl 2-(2-tert-butoxy-2-oxo-ethyl)indane-2-carboxylate

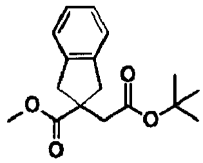

To a solution of methyl 2,3-dihydro-1H-indene-2-carboxylate (5 g, 28.3 mmol) in THF (100 mL) was added NaHMDS (21 mL, 42.5 mmol, 2M in THF) at -78 °C under argon and stirred at -78 °C for 1 h. Then tert-butyl 2-bromoacetate solution (6.4 mL, 42.5 mmol) in THF (30 mL) was added drop wise for 15 minutes at -78 °C and stirred at same temperature for 2 h. The reaction mixture was quenched with sat. ammonium chloride solution (50 mL) at -78 °C and allowed to stir at room temperature for 30 minutes. The organic layer was separated, aqueous layer was extracted with EtOAc (2x100 mL), and the combined organic layer was evaporated to get crude compound. The crude compound was triturated with n-pentane (50 mL) at -78°C and stirred at same temperature for 15 minutes. The resulting solid was filtered and dried under vacuum affording an off white solid (3.7 g, 45%). M/z = 313.0 $(M+Na)^+$.

h. 2-(2-tert-butoxy-2-oxo-ethyl)indane-2-carboxylic acid

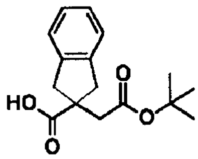

To a stirred solution of methyl 2-(2-(tert-butoxy)-2-oxoethyl)-2,3-dihydro-1H-indene-2-carboxylate (430 g, 1.48 mol) in THF (2.15 L) and ethanol (2.15 L) was added 0.5 M $LiOH.H_2O$ (6.8 L, 2.96 mol) drop wise at room temperature and stirred at same temperature for 2 h. The reaction mixture was evaporated to get the residue and the residue was diluted with $H_2O$ (1 L) and extracted with diethyl ether. The aqueous layer was acidified with IN HCl to pH 3-4. The resulting precipitate was filtered, washed with water, n-pentane and dried under vacuum affording a white solid (254.5 g, 62%). M/z 275.2 $(M-H)^-$. $^1H$ NMR (300 MHz, DMSO-$d_6$): $\delta$ 12.4 (1H, bs), 7.18-7.10 (4H, m), 3.39 (2H, d, J = 16.2 Hz), 2.92 (2H, d, J = 16.2 Hz), 2.64 (2H, s), 1.37 (9H, s).

i. Tert-butyl 2-[2-[[6-(3-hydroxypropoxy)-1,3-benzothiazol-2-yl]methylcarbamoyl]indan-2-yl]acetate

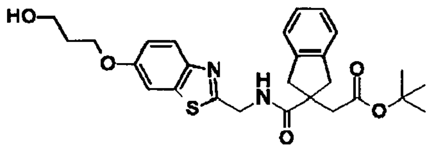

To a solution of 2-(2-(tert-butoxy)-2-oxoethyl)-2,3-dihydro-1H-indene-2-carboxylic acid (150 mg, 0.54 mmol) in DMF (6 mL) was added $Et_3N$ (0.2 mL, 1.62 mmol), EDC. HCl (125 mg, 0.65 mmol), HOBt (74 mg, 0.54 mmol) and 3-[[2-(aminomethyl)-1,3-benzothiazol-6-yl]oxy]propan-1-ol hydrochloride (164 mg, 0.59 mmol) at room temperature and stirred for 12 h. The reaction mixture was diluted with cold water (20 mL) and extracted with EtOAc (2 x 30 mL) and evaporated. The crude was purified by silica gel chromatography with 3-5% MeOH in DCM affording a yellow solid (125 mg, 46%). M/z = 497.2 $(M+H)^+$.

j. 2-[2-[[6-(3-hydroxypropoxy)-1,3-benzothiazol-2-yl]methylcarbamoyl]indan-2-yl]acetic acid

To a solution of tert-butyl 2-[2-[[6-(3-hydroxypropoxy)-1,3-benzothiazol-2-yl]methylcarbamoyl]indan-2-yl]acetate (110 mg, 0.22 mmol) in DCM (5 mL) was added TFA (2 mL) at 0 °C and stirred at room temperature for 2 h. The mixture was evaporated and the residue was triturated with diethyl ether (15 mL). The crude compound was purified by preparative HPLC [HPLC [SYMMETRY-C8 (300*19 mm), 7 u, Mobile phase: A: 0.1% Formic Acid in $H_2O$, B: MeCN] affording the title compound as an off white solid (20 mg, 20%). M/z 441.1 $(M+H)^+$. $^1H$ NMR (500 MHz, DMSO-$d_6$): δ 12.12 (1H, bs), 8.69 (1H, t, J = 6 Hz), 7.79 (1H, d, J = 9 Hz), 7.57 (1H, d, J = 2.5 Hz), 7.22-7.19 (2H, m), 7.15-7.13 (2H, m), 7.06 (1H, dd, J = 9 Hz, J = 2.5 Hz), 4.60 (2H, d, J = 6 Hz), 4.55 (1H, t, J = 5 Hz), 4.08 (2H, t, J = 6.5 Hz), 3.57 (2H, td, J = 6 Hz, J = 5 Hz), 3.44 (2H, d, J = 16 Hz), 3.00 (2H, d, J = 16 Hz), 2.73 (2H, s), 1.89-1.86 (2H, m).

**Example 2 2-[2- [(6-propoxy-1,3-benzothiazol-2-yl)methylcarbamoyl]indan-2-yl]acetic acid**

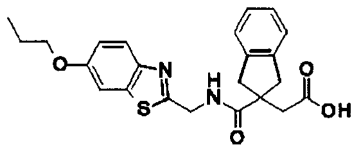

This was prepared in an analogous manner to Example 1 using 1-bromopropane in step-d. The title compound was isolated as white solid (37 mg, 38%). M/z 425.1 $(M+H)^+$. $^1H$ NMR (500 MHz, DMSO-$d_6$): δ 12.14 (1H, bs), 8.97 (1H, bs), 7.78 (1H, d, J = 9 Hz), 7.56 (1H, d, J = 2.5 Hz), 7.21-7.20 (2H, m), 7.14-7.12 (2H, m), 7.06 (1H, dd, J = 9.0 Hz, J= 2.5 Hz), 4.60 (2H, d, J = 5.5 Hz), 3.98 (2H, t, J = 6.5 Hz), 3.47 (2H, d, J = 16.5 Hz), 3.00 (2H, d, J = 16 Hz), 2.70 (2H, s), 1.77-1.72 (2H, m), 1.00 (3H, t, J = 7.5 Hz).

**Example 3 2-[2-[[5-[3-(dimethylamino)propoxy]-6-methoxy-1,3-benzothiazol-2-yl]methylcarbamoyl]indan-2-yl]acetic acid**

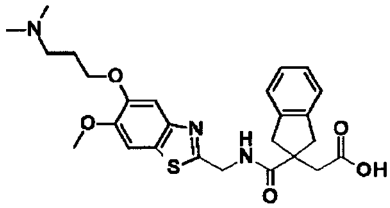

a. 4-bromo-5-methoxy-2-nitroaniline

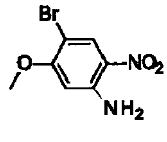

To a stirred solution of 5-methoxy-2-nitroaniline (100 g, 595 mmol) in acetonitrile (2.5 L) was added NBS (106 g, 595 mmol) portion wise at room temperature. The mixture was cooled to 0 °C and added TFA (46 mL, 595 mmol) drop wise for 30 minutes and allowed to stir at room temperature for 16 h. The reaction mixture was diluted with water (1 L) and adjusted the pH to ~ 8 with IN NaOH. The resulting precipitate was filtered, washed with water (500 mL) and dried under vacuum affording a yellow solid. (105 g, 72%). M/z 247 $(M+H)^+$.

b. 1-Bromo-4-iodo-2-methoxy-5-nitrobenzene

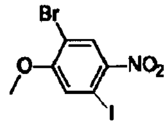

To a stirred solution of 4-bromo-5-methoxy-2-nitroaniline (50 g, 203 mmol) in acetonitrile (750 mL) was added concentrated $H_2SO_4$ (24 mL, 457 mmol) drop wise at -10 °C. Then $NaNO_2$ (28 g, 406 mmol) in water (175 mL) was added drop wise at -10 °C for 15 minutes and stirred at same temperature for 30 min. After that KI solution (135 g, 813 mmol) in water (175 mL) was added drop wise at -10 °C for 20 minutes and stirred at same temperature for 30 min. The reaction mixture was quenched with sodium metabisulphite solution (309 g, 1.62 mmol) in water (1.6 L) at -10 °C to 0 °C for 1h. Then water (1 L) was added and allowed to stir at room temperature for 30 minutes. The resulting precipitate was filtered, washed with water (1 L) and dried under vacuum affording a yellow solid. (60 g, 82%). M/z 357.8 $(M+H)^+$.

c. 5-Bromo-2-iodo-4-methoxyaniline

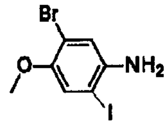

To a stirred solution of 1-bromo-4-iodo-2-methoxy-5-nitrobenzene (106 g, 296 mmol) in EtOH: $H_2O$ (800 mL: 200 mL) was added Fe (49.7 g, 890 mmol), $NH_4Cl$ (80 g, 1.48 mmol) at room temperature and stirred at 90 °C for 2 h. Then the reaction mixture was cooled to 60 °C, added additional amount of Fe (33 g, 593 mmol), $NH_4Cl$ (80 g 1.48 mmol) and stirred at 90 °C for 30 minutes. The reaction mixture was filtered through ciliate pad, washed the pad with methanol (1 L) and filtrate was concentrated to give residue. The residue was diluted with cold water (1 L) and adjusted the pH to ~8 with 1N NaOH. The resulting precipitate was filtered and dried under vacuum affording a light brown solid (90 g, 92%). M/z 327.8 $(M+H)^+$.

d. Tert-butyl N-[(5-bromo-6-methoxy-1,3-benzothiazol-2-yl)methyl]carbamate

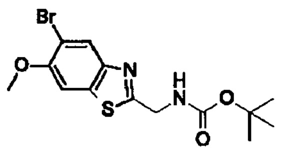

To a stirred solution of 5-bromo-2-iodo-4-methoxyaniline (50 g, 152 mmol) in acetonitrile (560 mL) was added tert-butyl(2-amino-2-thioxoethyl)carbamate (35 g, 183 mmol), CaO (17 g, 305 mmol) and degassed with argon for 20 minutes. Then $Pd_2(dba)_3$ (14 g, 15.2 mmol), dppf (25.4 g, 15.8 mmol) was added and purged with argon for further 5 minutes and the reaction mixture was stirred at 80 °C for 4 hour. The reaction mixture was filtered through celite pad and washed the pad with EtOAc (300 mL). The filtrate was washed with water and evaporated to get crude compound. The crude compound was dissolved in acetonitrile (200 mL), on standing for 1 hour solid was precipitated out. The resulting solid was filtered, washed with acetonitrile (50 mL) and dried under vacuum affording an off white solid (34 g, 60%). M/z 372.9 $(M+H)^+$.

e. Tert-butyl N-[[6-methoxy-5-(4,4,5,5-tetramethyl-1,3,2-dioxaborolan-2-yl)-1,3-benzothiazol-2-yl]methyl]carbamate

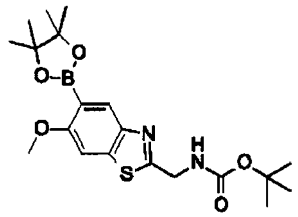

To a stirred solution of tert-butyl ((5-bromo-6-methoxybenzo[d]thiazol-2-yl)methyl)carbamate (5 g, 13.44 mmol) in dioxane (100 mL) was added BPin (6.8 g, 26.8 mmol), KOAc (4.6 g, 47.0 mmol) and purged with argon for 15 minutes. Then $Pd_2Cl_2$(dppf). DCM (1.1 g, 1.34 mmol) was added and purged with argon for further 5 minutes. The reaction mixture was heated at 100 °C for 16 h. The reaction mixture was filtered through celite pad and washed the pad with EtOAc (50 mL). The filtrate was washed with water, brine and evaporated affording a white solid (12 g, crude). M/z 339 $(M+H)^+$.

f. Tert-butyl N-[(5-hydroxy-6-methoxy-1,3-benzothiazol-2-yl)methyl]carbamate

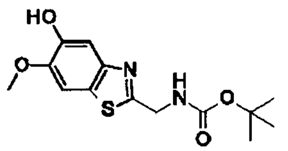

To a stirred solution of tert-butyl N-[[6-methoxy-5-(4,4,5,5-tetramethyl-1,3,2-dioxaborolan-2-yl)-1,3-benzothiazol-2-yl]methyl]carbamate (12 g, 35.5 mmol) in THF (180 mL) was added IN NaOH (35 mL, 35.5 mmol), 30% $H_2O_2$ (6.2 mL 81.6 mmol) at 0 °C and stirred at same temperature for 30 minutes. The reaction mixture was partitioned between water and EtOAc. The organic layer was separated washed with water, brine and evaporated to get crude compound. The crude compound was chromatographed on silica eluting with 30% EtOAc in pet ether affording an off white solid. (2.5 g 54%). M/z 311.0 $(M+H)^+$. $^1H$ NMR (500 MHz, $CDCl_3$): δ 7.50 (1H, s), 7.25 (1H, s), 5.76 (1H, s), 5.30 (1H, s), 4.68 (2H, d, J = 5.5 Hz), 3.97 (3H, s), 1.54 (9H, s). M/z 311.0 $(M+H)^+$.

g. Tert-butyl N-[[5-[3-(dimethylamino)propoxy]-6-methoxy-1,3-benzothiazol-2-yl]methyl]carbamate

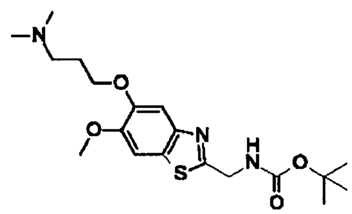

To a solution of tert-butyl N-[(5-hydroxy-6-methoxy-1,3-benzothiazol-2-yl)methyl]carbamate (750 mg, 2.41 mmol) in DMF (5 mL) was added $K_2CO_3$ (1 g, 7.25 mmol), 3-chloro-N,N-dimethylpropan-1-amine (355 mg, 2.90 mmol) at room temperature and heated at 80 °C for 4 h. The reaction mixture was diluted with water (25 mL) and extracted with EtOAc (2 x 30 mL). The combined organic layer was dried, filtered and evaporated affording a pale brown liquid (1 g, crude). M/z 395.8 $(M+H)^+$.

h. 3-[[2-(aminomethyl)-6-methoxy-1,3-benzothiazol-5-yl]oxy]-N,N-dimethyl-propan-1-amine hydrochloride

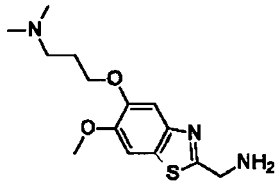

To a solution of tert-butyl N-[[5-[3-(dimethylamino)propoxy]-6-methoxy-1,3-benzothiazol-2-yl]methyl]carbamate (1 g, 2.53 mmol) in dioxane (5 mL) was added 4M HCl in dioxane (6 mL) at room temperature and stirred for 6 h. The reaction mixture was evaporated and the resulting residue was triturated with diethyl ether (25 mL) affording a pale yellow solid (0.92 g, crude). M/z 296.2 $(M+H)^+$.

i. Tert-butyl 2-[2-[[5-[3-(dimethylamino)propoxy]-6-methoxy-1,3-benzothiazol-2-yl]methylcarbamoyl]indan-2-yl] acetate

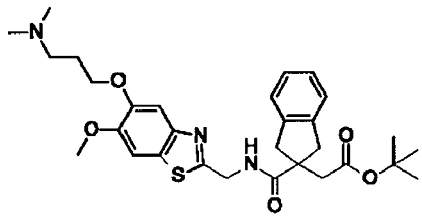

To a solution of 3-[[2-(aminomethyl)-6-methoxy-1,3-benzothiazol-5-yl]oxy]-N,N-dimethyl-propan-1-amine hydrochloride (450 mg, 1.52 mmol) in DMF (6 mL) was added $Et_3N$ (1.1 mL, 7.62 mmol) and stirred for 10 minutes. Then 2-(2-(tert-butoxy)-2-oxoethyl)-2,3-dihydro-1H-indene-2-carboxylic acid (463 mg, 1.67 mmol), EDC. HCl (440 mg, 2.28 mmol) and HOBt (210 mg, 1.52 mmol) was added at room temperature and stirred for 16 h. The reaction mixture was diluted with cold water (30 mL) and extracted with EtOAc (2 x 40 mL) and evaporated to get crude compound. The crude was chromatographed on silica eluting with 10-12% MeOH in DCM affording a yellow solid (310 mg, 56%). M/z 554.2 $(M+H)^+$.

j. 2-[2-[[5-[3-(dimethylamino)propoxy]-6-methoxy-1,3-benzothiazol-2-yl]methylcarbamoyl]indan-2-yl] acetic acid

To a solution of tert-butyl 2-[2-[[5-[3-(dimethylamino)propoxy]-6-methoxy-1,3-benzothiazol-2-yl]methylcarbamoyl]indan-2-yl]acetate (120 mg, 0.21 mmol) in DCM (5 mL) was added TFA (2 mL) at 0 °C and stirred at room temperature for 2 h. The mixture was evaporated and the residue was triturated with diethyl ether (15 mL). The crude compound was purified by preparative HPLC [YMC-TRIART(150 X 25 mm), 10 u, Mobile phase: A: 0.1% Formic Acid in $H_2O$, B: MeCN] affording the title compound as an off white solid (32 mg, 30%). M/z 498.1 $(M+H)^+$. $^1H$ NMR (500 MHz, DMSO-$d_6$): δ 9.00 (1H, bs), 7.55 (1H, s), 7.43 (1H, s), 7.21-7.20 (2H, m), 7.14-7.12 (2H, m), 4.60 (2H, d, J = 6 Hz), 4.04 (2H, t, J = 6.5 Hz), 3.81 (3H, s), 3.45 (2H, d, J = 16 Hz), 2.98 (2H, d, J = 16 Hz), 2.69 (2H, s), 2.40 (2H, t, J = 7 Hz), 2.17 (6H, s), 1.91-1.85 (2H, m).

**Example 4 2-[2-[[6-methoxy-5-[3-(trimethylammonio)propoxy]-1,3-benzothiazol-2-yl]methylcarbamoyl]indan-2-yl]acetate**

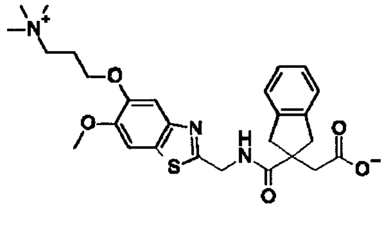

a. 3-[[2-[[[2-(2-tert-butoxy-2-oxo-ethyl)indane-2-carbonyl]amino]methyl]-6-methoxy-1,3-benzothiazol-5-yl]oxy]propyl-trimethyl-ammonium iodide

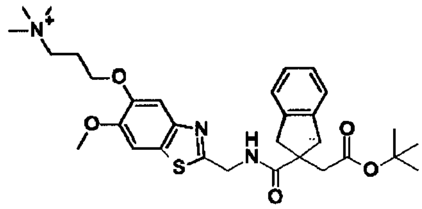

To a solution of tert-butyl 2-[2-[[5-[3-(dimethylamino)propoxy]-6-methoxy-1,3-benzothiazol-2-yl]methylcarbamoyl]indan-2-yl]acetate (200 mg, 0.36 mmol) in acetonitrile (5 mL) was added MeI (1 mL) at 0 °C and stirred at room temperature for 16 h. The mixture was evaporated and resulting residue was purified by silica gel chromatography eluting with 15-20% 7M $NH_3$/MeOH in DCM affording a pale yellow solid (100 mg, 49%). M/z 568.3 $(M)^+$.

b. 2-[2-[[6-methoxy-5-[3-(trimethylammonio)propoxy]-1,3-benzothiazol-2-yl]methylcarbamoyl]indan-2-yl]acetate

To a solution of 3-[[2-[[[2-(2-tert-butoxy-2-oxo-ethyl)indane-2-carbonyl]amino]methyl]-6-methoxy-1,3-benzothiazol-5-yl]oxy]propyl-trimethyl-ammonium iodide (90 mg, 0.15 mmol) in DCM (5 mL) was treated with TFA (1.5 mL) at 0 °C and stirred at room temperature for 4 h. The mixture was evaporated and the residue was triturated with diethyl ether (10 mL). The crude compound was purified by preparative HPLC [X-BRIDGE-C18 (150*30), 5 u, Mobile phase: A: 0.1% Formic Acid in $H_2O$, B: MeCN] affording the title compound as an off white solid (8.2 mg, 10%). M/z 512.3 $(M+H)^+$. $^1H$ NMR (500 MHz, DMSO-$d_6$): 12.27 (1H, bs), 7.64 (1H, s), 7.53 (1H, s), 7.17-7.15 (2H, m), 7.11-7.09 (2H, m), 4.61 (2H, d, J = 5.5 Hz), 4.12 (2H, t, J = 6 Hz), 3.82 (3H, s), 3.51-3.49 (2H, m), 3.40 (2H, d, J = 16 Hz), 3.10 (9H, s), 2.90 (2H, d, J = 16 Hz), 2.40 (2H, s), 2.24-2.21 (2H, m).

**Example 5 2-[2-[[6-methoxy-5-[2-[2-(trimethylammonio)ethoxy]ethoxy]-1,3-benzothiazol-2-yl]methylcarbamoyl]indan-2-yl]acetate**

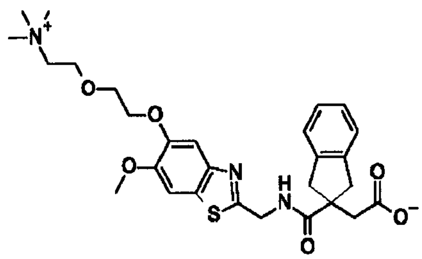

c. Tert-butyl N-[[5-[2-(2-chloroethoxy)ethoxy]-6-methoxy-1,3-benzothiazol-2-yl]methyl]carbamate

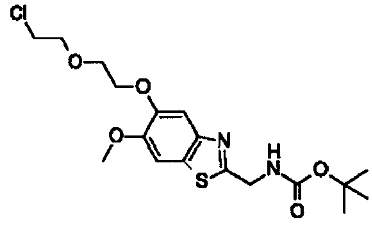

A solution of tert-butyl N-[(5-hydroxy-6-methoxy-1,3-benzothiazol-2-yl)methyl]carbamate (600 mg, 1.93 mmol)

in acetonitrile (10 mL) was added $Cs_2CO_3$ (692 mg, 2.12 mmol) and 1-chloro-2-(2-chloroethoxy)ethane (304 mg, 2.12 mmol) at room temperature. The mixture was heated at 70°C for 16 h, then filtered through celite pad and washed the pad with EtOAc (15 mL). The filtrate was concentrated and the residue was chromatographed on silica eluting with 25% EtOAc in petroleum ether affording an off white solid (250 mg, 32%). M/z 417.1 $(M+H)^+$

d. Tert-butyl N-[[5-[2-[2-(dimethylamino)ethoxy]ethoxy]-6-methoxy-1,3-benzothiazol-2-yl]methyl] carbamate

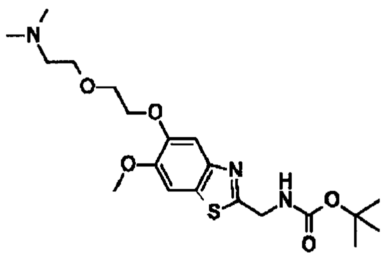

A solution of tert-butyl ((5-(2-(2-chloroethoxy)ethoxy)-6-methoxybenzo[d]thiazol-2-yl)methyl)carbamate (250 mg, 0.60 mmol) in acetone (5 mL) was added $Cs_2CO_3$ (293 mg, 0.90 mmol) and dimethylamine (2 mL, 2M in THF) at 0 °C. The mixture was heated at 90 °C in a sealed tube for 20 h. The reaction mixture was filtered through celite pad and washed the pad with EtOAc (15 mL). The filtrate was concentrated and the residue was chromatographed on silica eluting with 10-20% MeOH in DCM affording a pale yellow solid (210 mg, 84%). M/z 426.2 $(M+H)^+$

e. 2-(2-((2-(aminomethyl)-6-methoxybenzo[d]thiazol-5-yl)oxy)ethoxy)-N,N-dimethylethan-1-amine hydrochloride

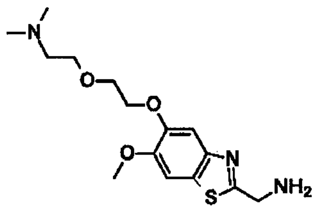

A solution of tert-butyl ((5-(2-(2-(dimethylamino)ethoxy)ethoxy)-6-methoxybenzo[d]thiazol-2-yl)methyl)carbamate (200 mg, 0.47 mmol) in dioxane (4 mL) was added 4M HCl in dioxane (5 mL) at room temperature. The mixture was stirred at room temperature for 4 h and concentrated under reduced pressure. The residue was triturated with diethyl ether (10 mL) affording an off white solid (180 mg, crude). M/z 326.1 $(M+H)^+$.

f. Tert-butyl 2-[2-[[5-[2-[2-(dimethylamino)ethoxy]ethoxy]-6-methoxy-1,3-benzothiazol-2-yl]methylcarbamoyl]indan-2-yl]acetate

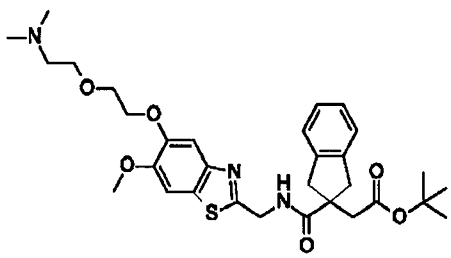

A solution of 2-(2-((2-(aminomethyl)-6-methoxybenzo[d]thiazol-5-yl)oxy)ethoxy)-N,N-dimethylethan-1-amine

hydrochloride (300 mg, 0.92 mmol) in DMF (8 mL) was added $Et_3N$ (0.4 mL, 2.76 mmol) and stirred for 10 minutes. Then 2-(2-(tert-butoxy)-2-oxoethyl)-2,3-dihydro-1H-indene-2-carboxylic acid (280 mg, 1.01 mmol) and T3P (0.9 mL, 1.38 mmol) was added at room temperature and stirred for 16 h. The reaction mixture was partitioned between water (15 mL) and EtOAc (30 mL). The organic layer was evaporated and resulting crude compound was chromatographed on silica eluting with 10%-20% MeOH in DCM affording an off white solid (200 mg, 38%). M/z 584.2 $(M+H)^+$.

g. 2-[2-[[2-[[[2-(2-tert-butoxy-2-oxo-ethyl)indane-2-carbonyl]amino]methyl]-6-methoxy-1,3-benzothiazol-5-yl]oxy]ethoxy]ethyl-trimethyl-ammonium

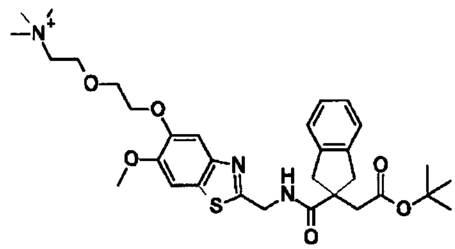

A solution of tert-butyl 2-(2-(((5-(2-(2-(dimethylamino)ethoxy)ethoxy)-6-methoxybenzo[d]thiazol-2-yl)methyl)carbamoyl)-2,3-dihydro-1H-inden-2-yl)acetate (200 mg, 0.34 mmol) in acetonitrile (5 mL) was added MeI (1 mL) at 0 °C and stirred at room temperature for 8 h. The mixture was evaporated and the residue was purified by preparative TLC eluting with 10% MeOH in DCM affording an off white solid (60 mg, 30%). M/z 598.1 $(M)^+$.

h. 2-[2-[[6-methoxy-5-[2-[2-(trimethylammonio)ethoxy]ethoxy]-1,3-benzothiazol-2-yl]methylcarbamoyl] indan-2-yl] acetate

A solution of 2-(2-((2-((2-(2-(tert-butoxy)-2-oxoethyl)-2,3-dihydro-1H-indene-2-carboxamido)methyl)-6-methoxybenzo[d]thiazol-5-yl)oxy)ethoxy)-N,N,N-trimethylethan-1-aminium (120 mg, 0.20 mmol) in DCM (5 mL) was treated with TFA (1 mL) at 0 °C and stirred at room temperature for 4 h. The mixture was evaporated and the residue was triturated with diethyl ether (10 mL). The crude compound was purified by preparative HPLC [X-BRIDGE-C18 (150*30), 5u, Mobile phase: A: 0.1% Formic Acid in $H_2O$, B: MeCN] and freeze dried affording the title product as an off white solid (46 mg, 43%). M/z 542.2 $(M+H)^+$. $^1H$ NMR (500 MHz, DMSO-$d_6$): δ 9.85 (1H, bs), 7.58 (1H, s), 7.49 (1H, s), 7.20-7.18 (2H, m), 7.13-7.11 (2H, m), 4.60 (2H, d, J = 5.0 Hz), 4.20 (2H, t, J = 4 Hz), 3.94 (2H, bs), 3.85 (2H, t, J = 4 Hz), 3.82 (3H, s), 3.53 (2H, t, J = 4.5 Hz), 3.43 (2H, d, J = 16 Hz), 3.10 (9H, s), 2.96 (2H, d, J = 16 Hz), 2.62 (2H, s).

**Example 6 2-[5,6-difluoro-2-[[6-methoxy-5-[2-[2-(trimethylammonio)ethoxy]ethoxy]-1,3-benzothiazol-2-yl]methylcarbamoyl]indan-2-yl]acetate**

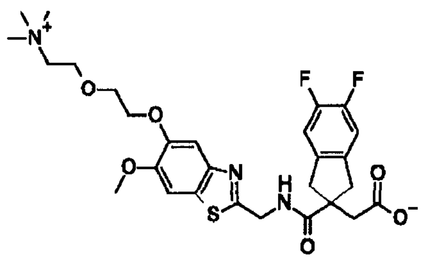

a. Dimethyl 4,5-difluorophthalate

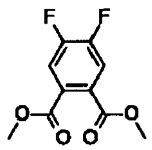

To an ice-cooled solution of 4,5-difluorophthalic acid (11.9 g, 58.9 mmol) in MeOH (250 mL) was added concentrated $H_2SO_4$ (40 mL, 0.75 mol) keeping the temperature <20 °C. The mixture was stirred at 65 °C for 4 h. The cooled reaction mixture was concentrated in vacuo, then the residue was cautiously added to EtOAc and aq. $NaHCO_3$. The aq. phase was extracted with EtOAc and the combined organic extracts were washed with aq. $NaHCO_3$, then brine, dried ($Na_2SO_4$), filtered and concentrated in vacuo to yield the title compound as a colourless oil (12.98 g, 96%). $^1$H NMR ($CDCl_3$) δ 7.56 (2H, *t, J* = 8.7 Hz), 3.91 (6H, s).

b. (4,5-Difluoro-1,2-phenylene)dimethanol

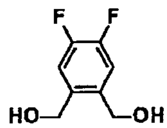

To an ice-cooled solution of lithium aluminium hydride (1M in THF, 226 mL, 0.226 mol) was added a solution of dimethyl 4,5-difluorophthalate (12.98 g, 56.4 mmol) in THF (100 mL) over 30 min keeping the temperature below 12 °C. The mixture was stirred in the ice bath for 30 min, then at RT for 1 h. The reaction mixture was cooled to 0 °C then, cautiously, water (8.5 mL), 15% aq. NaOH (8.5 mL) and water (26 mL) were added successively, keeping the temperature below 15 °C. Celite was added and the mixture stirred at RT for 1 h, then filtered through a celite pad, washing through with more THF. The filtrate was concentrated in vacuo to yield the title compound as a white solid (9.52 g, 97%). $^1$H NMR (d6-DMSO) δ 7.36 (2H, t, *J* = 10.1 Hz), 5.29 (2H, t, *J* = 5.5 Hz), 4.47 (4H, d, *J* = 5.4 Hz).

c. 1,2-Bis(bromomethyl)-4,5-difluorobenzene

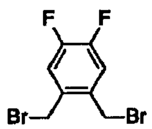

A mixture of (4,5-difluoro-1,2-phenylene)dimethanol (9.52 g, 54.7 mmol) and 48% hydrobromic acid (68.5 mL) was stirred at 110 °C for 1 h. The cooled reaction mixture was diluted with water and then extracted with $Et_2O$. The aq. phase was extracted with $Et_2O$ and the combined organic extracts were washed with water, then brine, dried ($Na_2SO_4$), filtered and concentrated in vacuo to leave a residue. FCC (1-10% EtOAc in hexane) to yield the title compound as a colourless oil (15.2 g, 93%). $^1$H NMR ($CDCl_3$) δ 7.20 (2H, *t, J* = 9.1 Hz), 4.55 (4H, s).

d. Diethyl 5,6-difluoro-1,3-dihydro-2*H*-indene-2,2-dicarboxylate

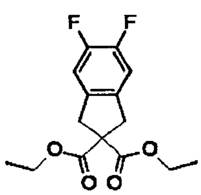

Sodium hydride (60% in oil, 4.46 g, 112 mmol) was added over 15 min to a mixture of 1,2-bis(bromomethyl)-4,5-difluorobenzene (15.2 g, 50.7 mmol) and diethyl malonate (9.74 g, 60.8 mmol) in THF (200 mL) keeping the temperature below 20 °C. The mixture was stirred at RT for 4 h, then saturated ammonium chloride was added. The mixture was concentrated in vacuo and then extracted twice with EtOAc. The combined organic extracts were washed with brine, dried ($Na_2SO_4$), filtered and concentrated in vacuo to leave a residue. FCC (5-25% EtOAc in hexane) yielded the title compound as a colourless oil (9.95 g, 66%). $^1$H NMR ($CDCl_3$) $\delta$ 6.97 (2H, *t, J* = 8.7 Hz), 4.21 (4H, q, *J* = 7.1 Hz), 3.52 (4H, s), 1.26 (6H, t, *J* = 7.1 Hz).

e. 5,6-Difluoro-2,3-dihydro-1*H*-indene-2-carboxylic acid

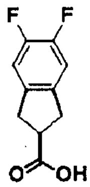

To a solution of diethyl 5,6-difluoro-1,3-dihydro-2*H*-indene-2,2-dicarboxylate (9.94g, 33.3 mmol) in dioxane (130 mL) was added water (130 mL) and concentrated HCl (140 mL). The mixture was refluxed for 23 h. The cooled reaction mixture was diluted with water and extracted with $Et_2O$ ($\times$3). The combined organic extracts were washed with water, then brine, dried ($Na_2SO_4$), filtered and concentrated in vacuo to yield the title compound as a colourless solid (6.6 g, quant.). M/z 197 $(M-H)^-$.

f. Methyl 5,6-difluoro-2,3-dihydro-1*H*-indene-2-carboxylate

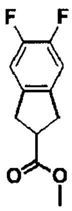

To an ice-cooled solution of 5,6-difluoro-2,3-dihydro-1*H*-indene-2-carboxylic acid (6.6 g, 33.3 mmol) in MeOH (200 mL) was added concentrated $H_2SO_4$ (40 mL, 0.75 mol) keeping the temperature <20 °C. The mixture was stirred at 65 °C for 1 h. The cooled reaction mixture was concentrated in vacuo, then the residue was cautiously added to EtOAc and aq. $NaHCO_3$. The aq. phase was extracted with more EtOAc and the combined organic extracts were washed with brine, dried ($Na_2SO_4$), filtered and concentrated in vacuo to leave a residue. FCC (5-25% EtOAc in hexane) yielded the title compound as a pale yellow solid (5.97 g, 84%). $^1$H NMR ($CDCl_3$) $\delta$ 6.98 (2H, *t, J* = 8.8 Hz), 3.73 (3H, s), 3.39 (1H, m), 3.24-3.12 (4H, m).

g. Methyl 2-(2-(*tert*-butoxy)-2-oxoethyl)-5,6-difluoro-2,3-dihydro-1*H*-indene-2-carboxylate

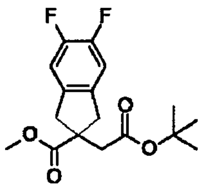

To a solution of methyl 5,6-difluoro-2,3-dihydro-1*H*-indene-2-carboxylate (5.97 g, 28.2 mmol) in THF (120 mL), cooled to -78 °C, was added sodium bis(trimethylsilyl)amide (1M in THF, 42.2 mL, 42.2 mol) over 15 min. The mixture

was stirred at -78 °C for 45 min then a solution of tert-butyl bromoacetate (8.24 g, 42.2 mmol) in THF (15 mL) was added over 10 min. The reaction mixture was allowed to warm to -10 °C over 1 h. Saturated ammonium chloride was added and the mixture was concentrated under reduced pressure. The residue was extracted twice with EtOAc and the combined organic extracts were washed with brine, dried ($Na_2SO_4$), filtered and concentrated in vacuo to leave a residue. FCC (5-20% EtOAc in hexane) yielded the title compound as a yellow gum (8.78 g, 96%). $^1$H NMR ($CDCl_3$) δ 6.96 (2H, *t, J* = 8.9 Hz), 3.72 (3H, s), 3.47 (2H, d, *J* = 16.2 Hz), 2.90 (2H, d, *J* = 16.2 Hz), 2.71 (2H, s), 1.42 (9H, s).

h. 2-[(tert-butoxy)carbonyl]-5,6-difluoro-2,3-dihydro-1H-indene-2-carboxylic acid

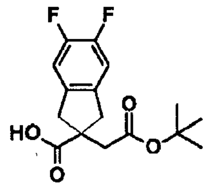

To a solution of methyl 2-(2-(*tert*-butoxy)-2-oxoethyl)-5,6-difluoro-2,3-dihydro-1*H*-indene-2-carboxylate (0.834 g, 2.56 mmol) in THF (25 mL) and MeOH (10 mL) was added lithium hydroxide (0.5M in water, 10.2 mL, 5.1 mmol). The mixture was stirred at RT for 2.5 h, then concentrated in vacuo. The residual solution was layered with EtOAc and acidified by addition of 6M HCl. The aq. phase was extracted with more EtOAc and the combined organic extracts were washed with brine, dried ($Na_2SO_4$), filtered and concentrated in vacuo to leave a residue. FCC (2-6% MeOH in DCM) yielded the title compound as a cream solid (0.59 g, 74%). $^1$H NMR (d6-DMSO) δ 12.47 (1H, bs), 7.26 (2H, t, *J* = 9.2 Hz), 3.33 (2H, d, *J* = 16.4 Hz), 2.91 (2H, d, *J* = 16.4 Hz), 2.67 (2H, s), 1.37 (9H, s). M/z 311 $(M-H)^-$.

i. 2-[5,6-difluoro-2-[[6-methoxy-5-[2-[2-(trimethylammonio)ethoxy]ethoxy]-1,3-benzothiazol-2-yl]methylcarbamoyl]indan-2-yl]acetate

This was prepared in an analogous manner to Example 5 using 2-[(tert-butoxy)carbonyl]-5,6-difluoro-2,3-dihydro-1H-indene-2-carboxylic acid in step-d. The title compound was isolated as an orange solid (58.5 mg). M/z 578.5 $(M+H)^+$. $^1$H NMR (500 MHz, DMSO-$d_6$): δ 13.05 (1H, bs), 7.60 (1H, s), 7.61 (1H, s), 7.22-7.21 (1H, m), 7.17-7.14 (1H, m), 4.62 (2H, d, J = 6 Hz), 4.21 (2H, m), 3.93 (2H, m), 3.87 (2H, s), 3.82 (3H, s), 3.58 (2H, m), 3.36 (2H, m), 3.10 (9H, s), 2.89-2.81 (2H, m), 2.32 (2H, m).

**Example 7 2-[5,6-difluoro-2-[[6-methoxy-5-[2-(trimethylammonio)ethoxy]-1,3-benzothiazol-2-yl]methylcarbamoyl]indan-2-yl]acetate**

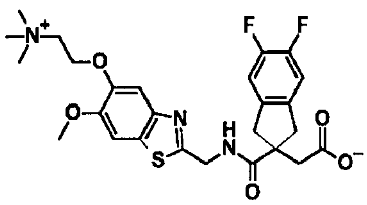

This was prepared in an analogous manner to Example 5 using in 2-Chloro-N,N-dimethylethylamine hydrochloride in step-a and 2-[(tert-butoxy)carbonyl]-5,6-difluoro-2,3-dihydro-1H-indene-2-carboxylic acid in step-d. The title compound was isolated as a white solid (13 mg, 12%). M/z 534.3 $(M+H)^+$. $^1$H NMR (500 MHz, DMSO-$d_6$): δ 13.16 (1H, bs), 7.69 (1H, s), 7.63 (1H, s), 7.22-7.21 (1H, m), 7.17-7.14 (1H, m), 4.61 (2H, m), 4.53 (2H, m), 3.82 (5H, m), 3.32 (2H, m), 3.21 (9H, s), 2.89-2.81 (2H, m), 2.32 (2H, m).

**Example 8: LasB Inhibitory Activity Measurements**

The relevance of LasB to PA infection is illustrated in Figure 1, which shows lung burden in a rat model of chronic lung infection following infection with WT PA (which expresses LasB) and a mutant form of PA (Δ*lasB* PA) in

which LasB is not expressed. It can be clearly seen in that following infection, whereas a wild type strain is able to persist at least for 14 days, a LasB deficient strain was not able to persist beyond day 5.

The relevance of LasB to PA biofilm development is shown in Figure 2. Biofilms formed after 3 days by both PA26 wt and PA26 *lasB* deletion strains were investigated by confocal imaging and subsequent analysis (with Comstat software). This study demonstrated that biofilms formed by the PA26 *lasB* deletion strain were highly reduced in thickness and biomass compared to the wt strain, demonstrating the essential role of LasB in PA biofilm development (Asterisks ** and *** indicates statistical difference for $P \leq 0.05$, $P \leq 0.01$; respectively).

Experiments were therefore conducted (1) to measure the potency of inhibition of compounds of the invention against purified *Pseudomonas aeruginosa* LasB enzyme and also experiments were conducted (2) to measure the ability of compounds of the invention to inhibit LasB-catalysed elastin degradation. The first assay uses a commercial fluorescent synthetic peptide and purified LasB enzyme. The LasB hydrolysis kinetics are measured allowing the determination of the IC50 and Ki of the inhibitors; the second is a more physiological assay using dialysed *Pseudomonas aeruginosa* supernatant as source of enzyme, plus its natural substrate Elastin. It is an "end point assay" that determines the percentage of LasB inhibition by each compound for one particular time point and inhibitor concentration. Technical details are described below:

*Fluorometric assay to determine Ki*

This assay uses commercially available substrate (Abz-Ala-Gly-Leu-Ala-p-Nitro-Benzyl-Amide (Ex: 340 nm, Em: 415 nm) from Peptide International) and purified LasB protein from *P. aeruginosa* (provided by Merck or Charles River Laboratories). It is performed to determine LasB elastase activity and assess compound inhibition in 96-well plate format. All compounds of Formula (I) were assessed using the method described below.

Method: 10 to 140 ng/ml purified LasB is incubated with 250 μM Abz-Ala-Gly-Leu-Ala-p-Nitro-Benzyl-Amide in 50 mM Tris-HCl pH 7.4, 2.5 mM CaCl2, 0.01% of Triton X100 at 37°C. LasB activity (corresponding to fluorescence emission induced by substrate hydrolysis) is measured over 30 min at 37°C with a fluorescence plate reader such as the Perkin Elmer Envision or similar. Different range of inhibitor concentrations are routinely assessed depending of inhibitor potency from 0.0016 to 200 μM (2-fold dilutions series) in order to determine IC50.

The equation used to calculate the Ki from IC50 is: Ki = IC50 / (1+([S]/Km)) where [S] = 250 μM and Km = 214 μM.

*Elastin assay to determine % inhibition*

The Elastin assay uses as source of enzyme dialysed supernatant from *P. aeruginosa* PAO1 and the Elastin Congo-Red as substrate. The natural LasB substrate, elastin, is complexed with the congo-red dye (Elastin Congo-Red, ECR). The elastolysis activity from the culture supernatant will degrade elastin and release the congo-red dye into the supernatant. This red dye release can be measured with a spectrophotometer.

All compounds of Formula (I) were assessed using the method described below.

Method: To determine LasB elastase activity and assess compound inhibition, an overnight culture of *P. aeruginosa* strain PAO1 is diluted in LB medium. After reaching an $OD_{600nm}$ of 0.6, this culture is diluted and incubated for additional 18-24 hours in a shaking incubator. Culture supernatants are recovered by centrifugation and filtrated through a 0.22 μM filter. These supernatants are dialysed (filtration molecules < 20kDa) into a 50 mM Tris-HCl pH 7.4, 2.5 mM $CaCl_2$ solution at 4°C under agitation for 24 hours. Supernatant dialysed is then mixed volume/volume with the ECR suspension (20 mg/mL of ECR in 100 mM Tris-HCl pH 7.4 buffer supplemented with 1 mM CaCl2) supplemented with Triton X100 (final concentration of 0.01%) in presence of DMSO (positive control) and/or different concentrations of compound (routinely 50 to 1.56 μM). As a negative control, the dialysed supernatant is replaced by Tris-HCl solution (50 mM Tris-HCl pH 7.4, 2.5 mM $CaCl_2$). The mixed reaction is then incubated overnight in a 37°C shaking incubator. The reaction supernatant is recovered by centrifugation and the release of congo-red is measured by its absorbance at 495 nm ($OD_{495nm}$).

Percentage inhibition is determined using the following equation:

$$((OD_{495nm}\text{ value of positive control} - OD_{495nm}\text{ value of negative control}) - (OD_{495nm}\text{ value of treated supernatant} - OD_{495nm}\text{ value of negative control})) / (OD_{495nm}\text{ value of positive control} - OD_{495nm}\text{ value of negative control}) \times 100.$$

Results are shown in the Table below and categorised into A, B and C for both assays. The Ki values are grouped as A (Ki = 0.00 to 0.050 μM), B (Ki = 0.05 to 0.1 μM) and C (Ki = 0.1 to 10.00 μM). Similarly, for the elastase

hydrolysis assay, values are grouped into A (>75% inhibition), B (60 to 75% inhibition) and C (10 to 60% inhibition) all at 25 μM inhibitor concentration. (n.d. not determined).

| Example | Ki (μM) | Elastin hydrolysis % inhibition @ 50 / 25 μM inhibitor concentration |
|---|---|---|
| **1** | B | A |
| **2** | B | A |
| **3** | B | A |
| **4** | A | A |
| **5** | B | B |
| **6** | A | A |
| **7** | B | B |

**Claims**

**1.** A compound which is an indane according to Formula (I), or a pharmaceutically acceptable salt thereof,

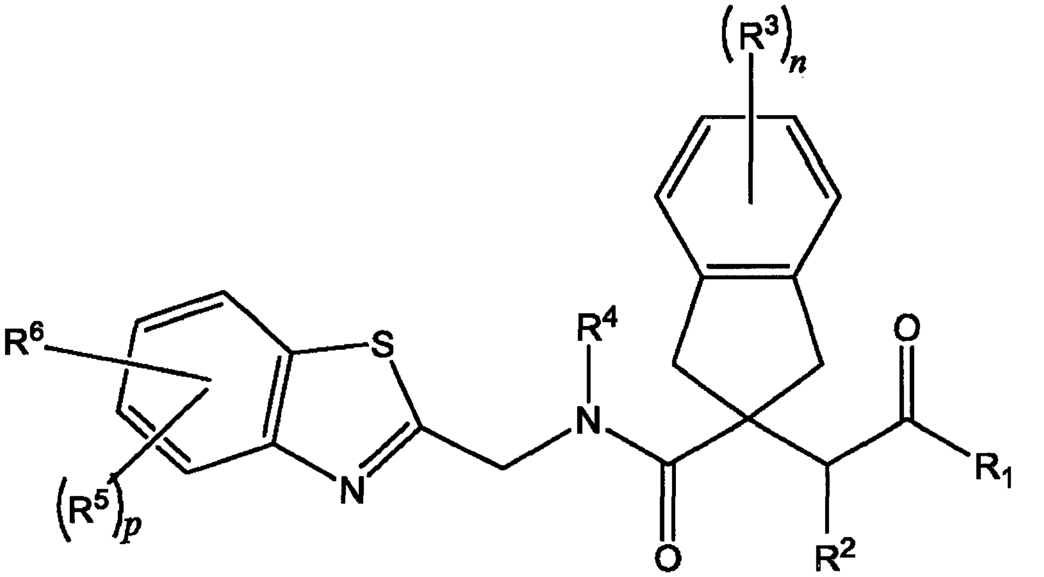

[FORMULA (I)]

wherein

• $R^1$ is selected from:

- NHOH, -OH, $-OR^{1a}$ and $-OCH_2OC(O)R^{1a}$, wherein $R^{1a}$ is selected from an unsubstituted $C_1$ to $C_4$ alkyl group and phenyl; and
- where the compound of Formula (I) contains a positively charged nitrogen atom, $R^1$ may be O, such that the compound forms a zwitterion;

• $R^2$ is selected from H and unsubstituted $C_1$ to $C_2$ alkyl;
• each $R^3$ group is independently selected from halogen, -OH, $-NH_2$, methyl and $-CF_3$;
• *n* is an integer from 0 to 4;
• $R^4$ is selected from H and unsubstituted $C_1$ to $C_2$ alkyl;
• $R^6$ is $C_2$ to $C_4$ alkoxy which is unsubstituted or is substituted with a group selected from -OH; $-NR^{10}R^{11}$; $-N^+R^{10}R^{11}R^{12}$; $-OR^{6a}$ and $-NR^{10}R^{6a}$, wherein $R^{6a}$ is a $C_1$ to $C_3$ alkyl group which is unsubstituted or substituted

with a group selected from OH; $-NR^{10}R^{11}$; $-N^+R^{10}R^{11}R^{12}$; $-NR^{10}NR^{11}R^{12}$; $-NR^{10}N^+R^{11}R^{12}R^{13}$; $-N^+R^{10}R^{11}NR^{12}R^{13}$;
$-NR^{10}C(NR^{11})NR^{12}R^{13}$; $-NR^{10}C(N^+R^{11}R^{12})NR^{13}R^{14}$; $-C(NR^{10})NR^{11}R^{12}$; and $-C(N^+R^{10}R^{11})NR^{12}R^{13}$;

- *p* is 0 or 1;
- $R^5$ is selected from -OMe, -OH, halogen, $-NR^{10}R^{11}$; $-N^+R^{10}R^{11}R^{12}$, $-CF_3$; and
- $R^{10}$, $R^{11}$, $R^{12}$ $R^{13}$ and $R^{14}$ are independently H or methyl;

with the proviso that the indane of Formula (I) is other than:

2-(2-(((4-ethoxybenzo[d]thiazol-2-yl)methyl)carbamoyl)-2,3-dihydro-1H-inden-2-yl)acetic acid;
2-[2-[(6-ethoxy-1,3-benzothiazol-2-yl)methylcarbamoyl] indan-2-yl] acetic acid;
2-[2-[[6-(2-hydroxyethoxy)-1,3-benzothiazol-2-yl]methylcarbamoyl]indan-2-yl]acetic acid;
2-[2-[[6-[2-(dimethylamino)ethoxy] -1,3-benzothiazol-2-yl]methylcarbamoyl] indan-2-yl]acetic acid;
2-[2-[[6-[2-(trimethylammonio)ethoxy]-1,3-benzothiazol-2-yl]methylcarbamoyl]indan-2-yl]acetate;
2-[2-[[5-[2-(dimethylamino)ethoxy]-1,3-benzothiazol-2-yl]methylcarbamoyl]indan-2-yl]acetic acid;
2-[2-[[5-[2-(trimethylammonio)ethoxy]-1,3-benzothiazol-2-yl]methylcarbamoyl]indan-2-yl]acetate;
2-(2-(((5-(3-(dimethylamino)propoxy)-6-methoxybenzo[d]thiazol-2-yl)methyl)carbamoyl)-5,6-difluoro-2,3-dihydro-1H-inden-2-yl)acetic acid;
2-(5,6-difluoro-2-(((6-methoxy-5-(3-(trimethylammonio)propoxy)benzo[d]thiazol-2-yl)methyl)carbamoyl)-2,3-dihydro-1H-inden-2-yl)acetate; and
2-(2-(((5-(2-(dimethylamino)ethoxy)-6-methoxybenzo[d]thiazol-2-yl)methyl)carbamoyl)-2,3-dihydro-1H-inden-2-yl)acetic acid.

**2.** A compound according to claim 1, wherein $R^1$ is selected from -OH and -NHOH, or where the compound of Formula (I) contains a positively charged nitrogen atom, $R^1$ may be O, such that the compound forms a zwitterion.

**3.** A compound according to claim 1 or claim 2, wherein

- $R^2$ is H; and
- $R^4$ is H.

**4.** A compound according to any one of the preceding claims, wherein n is an integer from 0 to 2 and each $R^3$ group is halogen, preferably fluorine.

**5.** A compound according to any one of claims 1 to 4, wherein $R^6$ is $C_2$ to $C_4$ alkoxy which is unsubstituted or is substituted with a group selected from -OH; $-NR^{10}R^{11}$;$-N^+R^{10}R^{11}R^{12}$; and $-OR^{6a}$, wherein $R^{6a}$ is a $C_1$ to $C_3$ alkyl group which is unsubstituted or substituted with a group selected from OH; $-NR^{10}R^{11}$; and $-N^+R^{10}R^{11}R^{12}$.

**6.** A compound according to any one of the preceding claims, wherein *p* is 1; and $R^6$ is $C_2$ to $C_4$ alkoxy which is substituted with a group selected from $-NR^{10}R^{11}$; $-N^+R^{10}R^{11}R^{12}$; and $-OR^{6a}$, wherein $R^{6a}$ is a $C_1$ to $C_3$ alkyl group which is unsubstituted or substituted with a group selected from $-NR^{10}R^{11}$; and $-N^+R^{10}R^{11}R^{12}$.

**7.** A compound according to any one of claims 1 to 4, wherein $R^6$ is $C_2$ to $C_4$ alkoxy which is substituted with a group selected from $-OR^{6a}$ and $-NR^{10}R^{6a}$, wherein $R^{6a}$ is a $C_1$ to $C_3$ alkyl group which is unsubstituted or substituted with a group selected from OH;$-NR^{10}R^{11}$; $-N^+R^{10}R^{11}R^{12}$; $-NR^{10}NR^{11}R^{12}$; $-NR^{10}N^+R^{11}R^{12}R^{13}$; $-N^+R^{10}R^{11}NR^{12}R^{13}$; $-NR^{10}C(NR^{11})NR^{12}R^{13}$; $-NR^{10}C(N^+R^{11}R^{12})NR^{13}R^{14}$; $-C(NR^{10})NR^{11}R^{12}$; and $-C(N^+R^{10}R^{11})NR^{12}R^{13}$, preferably $R^6$ is $C_2$ to $C_4$ alkoxy which is substituted with a group $-OR^{6a}$, wherein $R^{6a}$ is a $C_1$ to $C_3$ alkyl group which is unsubstituted or substituted with a group selected from OH; $-NR^{10}R^{11}$; and $-N^+R^{10}R^{11}R^{12}$.

**8.** A compound according to claim 1 which is 2-[5,6-difluoro-2-[[6-methoxy-5-[2-[2-(trimethylammonio)ethoxy]ethoxy]-1,3-benzothiazol-2-yl]methylcarbamoyl]indan-2-yl]acetate or a pharmaceutically acceptable salt thereof.

**9.** A compound according to claim 1 which is 2-[2-[[6-methoxy-5-[3-(trimethylammonio)propoxy]-1,3-benzothiazol-2-yl]methylcarbamoyl]indan-2-yl]acetate or a pharmaceutically acceptable salt thereof.

**10.** A compound according to claim 1 which is 2-[2-[[6-methoxy-5-[2-[2-(trimethylammonio)ethoxy]ethoxy]-1,3-benzothiazol-2-yl]methylcarbamoyl]indan-2-yl]acetate or a pharmaceutically acceptable salt thereof.

**11.** A compound according to claim 1 which is
2-[2-[[6-(3-hydroxypropoxy)-1,3-benzothiazol-2-yl]methylcarbamoyl]indan-2-yl]acetic acid;
2-[2-[(6-propoxy-1,3-benzothiazol-2-yl)methylcarbamoyl]indan-2-yl]acetic acid;
2-[2-[[5-[3-(dimethylamino)propoxy]-6-methoxy-1,3-benzothiazol-2-yl]methylcarbamoyl]indan-2-yl]acetic acid;
2-[5,6-difluoro-2-[[6-methoxy-5-[2-(trimethylammonio)ethoxy]-1,3-benzothiazol-2-yl]methylcarbamoyl]indan-2-yl]acetate;
2-(2-(((5-(4-(dimethylamino)butoxy)-6-methoxybenzo[d]thiazol-2-yl)methyl)carbamoyl)-2,3-dihydro-1H-inden-2-yl)acetic acid;
2-(2-(((6-methoxy-5-(4-(trimethylammonio)butoxy)benzo[d]thiazol-2-yl)methyl)carbamoyl)-2,3-dihydro-1H-inden-2-yl)acetate;
or a pharmaceutically acceptable salt thereof.

**12.** A pharmaceutical composition comprising (i) a compound according to any one of the preceding claims and (ii) at least one pharmaceutically acceptable carrier or diluent, and optionally further comprising (iii) an antibiotic agent; wherein preferably the antibiotic agent is selected from tobramycin, neomycin, streptomycin, gentamycin, ceftazidime, ticarcillin, piperacillin, tazobactam, imipenem, meropenem, rifampicin, ciprofloxacin, amikacin, colistin, aztreonam and levofloxacin.

**13.** A combination of (i) a compound according to any one of claims 1 to 11 and (ii) an antibiotic agent,
wherein preferably the antibiotic agent is selected from tobramycin, neomycin, streptomycin, gentamycin, ceftazidime, ticarcillin, piperacillin, tazobactam, imipenem, meropenem, rifampicin, ciprofloxacin, amikacin, colistin, aztreonam and levofloxacin.

**14.** A compound according to any one of claims 1 to 11; a composition according to claim 12 or a combination according to claim 13 for use in medicine.

**15.** A compound according to any one of claims 1 to 11; a composition according to claim 12 or a combination according to claim 13 for use in treating or preventing bacterial infection in a subject;
wherein preferably

- the bacterial infection is caused by *Bacillus, Pseudomonas, Staphylococcus, Streptococcus, Listeria, Burkholderia or Escherichia;*
- the compound for use, composition for use or combination for use is for use in the treatment or prevention of pneumonia; and/or
- the subject suffers from cystic fibrosis.

Europäisches Patentamt
European Patent Office
Office européen des brevets

# EUROPEAN SEARCH REPORT

Application Number
EP 18 29 0106

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | G. R. A. CATHCART ET AL: "Novel Inhibitors of the Pseudomonas aeruginosa Virulence Factor LasB: a Potential Therapeutic Approach for the Attenuation of Virulence Mechanisms in Pseudomonal Infection", ANTIMICROBIAL AGENTS AND CHEMOTHERAPY, vol. 55, no. 6, 1 June 2011 (2011-06-01), pages 2670-2678, XP055156158, ISSN: 0066-4804, DOI: 10.1128/AAC.00776-10 | 1-7, 12-15 | INV. C07D277/64 A61P25/00 A61P31/04 A61K31/428 |
| A | * figure 1; table 1 * | 8-11 | |
| A | WO 2014/083033 A1 (BAYER CROPSIENCE AG [DE]) 5 June 2014 (2014-06-05) * claim 1; compounds I-1 * | 1-15 | |
| A | US 2012/122764 A1 (KARKI RAJESHRI GANESH [US] ET AL) 17 May 2012 (2012-05-17) * precursor of example 11-1; claims 1-18 * | 1-15 | |

TECHNICAL FIELDS SEARCHED (IPC)

C07D
A61P

The present search report has been drawn up for all claims

2

| Place of search | Date of completion of the search | Examiner |
|---|---|---|
| Munich | 16 November 2018 | Sáez Díaz, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 18 29 0106

This annex lists the patent family members relating to the patent documents cited in the above-mentioned European search report. The members are as contained in the European Patent Office EDP file on
The European Patent Office is in no way liable for these particulars which are merely given for the purpose of information.

16-11-2018

| Patent document cited in search report | Publication date | Patent family member(s) | Publication date |
|---|---|---|---|
| WO 2014083033 A1 | 05-06-2014 | AR 093674 A1 | 17-06-2015 |
| | | BR 112015012054 A2 | 11-07-2017 |
| | | CA 2892702 A1 | 05-06-2014 |
| | | CN 104837351 A | 12-08-2015 |
| | | EA 201500584 A1 | 30-11-2015 |
| | | EP 2925137 A1 | 07-10-2015 |
| | | JP 6367214 B2 | 01-08-2018 |
| | | JP 2016503431 A | 04-02-2016 |
| | | UA 117820 C2 | 10-10-2018 |
| | | US 2015282483 A1 | 08-10-2015 |
| | | WO 2014083033 A1 | 05-06-2014 |
| US 2012122764 A1 | 17-05-2012 | AR 083872 A1 | 27-03-2013 |
| | | CN 103249715 A | 14-08-2013 |
| | | EP 2640689 A1 | 25-09-2013 |
| | | JP 2014507372 A | 27-03-2014 |
| | | TW 201300353 A | 01-01-2013 |
| | | US 2012122764 A1 | 17-05-2012 |
| | | WO 2012065953 A1 | 24-05-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 200629153 A, Bell, I.M. and Stump, C.A.
- WO 2008151211 A
- US 2006223830 A
- WO 2006125511 A


### Non-patent literature cited in the description


- **ROBINSON, R.P. et al.** *Bioorganic and Medicinal Chemistry Letters,* 1996, 1719
- **GANESHPURKAR, A. et al.** *Current Organic Syntheses,* 2018, vol. 15, 154-165
- **DING, C. et al.** *Bioorg. Med. Chem. Lett,* 2017, vol. 25, 27-37
- **SETH, S.** A Comprehensive Review on Recent advances in Synthesis & Pharmacotherapeutic potential of Benzothiazoles. *Anti-Inflammatory & Anti-Allergy Agents in Medicinal Chemistry,* 2015, vol. 14, 98-112
- **TAKAGI, K. et al.** *Chemistry Letters,* 1987, vol. 16, 839-840
- **DESROY, N. et al.** *Journal of Medicinal Chemistry,* 2013, vol. 56, 1418-1430
- **MALINGER, A. et al.** *Journal of Medicinal Chemistry,* 2016, vol. 59, 1078-1101
- **LIU, J. et al.** *Tetrahedron Letters,* 2017, vol. 58, 1470-1473
- **VALEUR, E. ; BRADLEY, M.** *Chem. Soc. Rev.,* 2008, vol. 28, 606-631